# EUROPÄISCHE PATENTSCHRIFT

(11) **EP 3 359 948 B1**
(45) Veröffentlichungstag und Bekanntmachung des Hinweises auf die Patenterteilung: **27.02.2019**
(21) Anmeldenummer: 15828640.1
(22) Anmeldetag: 09.12.2015
(51) Int. Cl.: G01N 21/17, G01N 21/552, A61B 5/145, A61B 5/1455, G01N 33/49, G01N 21/63, A61B 5/00, A61B 5/1495

(54) **VORRICHTUNG UND VERFAHREN ZUM ANALYSIEREN EINES STOFFS**
APPARATUS AND METHOD FOR ANALYZING A MATERIAL
DISPOSITIF ET PROCÉDÉ POUR ANALYSER UNE SUBSTANCE

(43) Veröffentlichungstag der Anmeldung: 15.08.2018
(73) Patentinhaber: Diamontech GmbH, 10245 Berlin (DE)
(72) Erfinder: BAUER, Alexander, 61440 Oberursel (DE); HERTZBERG, Otto, 60318 Frankfurt am Main (DE); LUBINSKI, Thorsten, 10245 Berlin (DE)
(74) Vertreter: Lucke, Andreas
(86) Internationale Anmeldenummer: PCT/DE2015/200532
(87) Internationale Veröffentlichungsnummer: WO 2017/097276

(56) Entgegenhaltungen:
- EP-A1- 1 048 265
- DE-A1- 3 146 700
- DE-B3-102005 048 807
- DE-B3-102014 108 424
- DE-C1- 4 446 390
- US-A1- 2013 286 397

## Beschreibung

Das vorliegende Schutzrecht bezieht sich auf eine Vorrichtung und ein Verfahren zum Analysieren eines Stoffs. Die hier beschriebene Vorrichtung und das hier beschriebene Verfahren können zum Beispiel zur Analyse von tierischem oder menschlichem Gewebe, in einer Ausführungsform zur Messung von Glukose bzw. Blutzucker, genutzt werden.

Bekannte Verfahren zum Analysieren eines Stoffs, insbesondere zum Messen von Blutzucker, sind beispielsweise in den folgenden Druckschriften beschrieben:
- Guo et al.: "Noninvasive glucose detection in human skin - using wavelength modulated differential laser photothermal radiometry", Biomedical Optics Express, Vol, 3, 2012, No. 11,
- Uemura et al.:"Non-invasive blood glucose measurement by Fourier transform infrared spectroscopic analysis through the mucous membrane of the lip: application of a chalcogenide optical fiber system", Front Med Biol Eng. 1999; 9(2): 137-153,
- Farahi et al.: "Pump probe photothermal spectroscopy using quantum cascade lasers", J. Phys. D. Appl. Phys. 45 (2012) und
- M. Fujinami et al.: "Highly sensitive detection of molecules at the liquid/liquid interface using total internal reflection-optical beam deflection based an photothermal spectroscopy", Rev. Sci. Instrum., Vol. 74, Number 1 (2003).
- (1) von Lilienfeld-Toal, H. Weidenmüller, M. Xhelaj, A. Mäntele, W. A Novel Approach to Non- Invasive Glucose Measurement by Mid-Infrared Spectroscopy: The Combination of Quantum Cascade Lasers (QCL) and Photoacoustic Detection Vibrational Spectroscopy, 38:209-215, 2005.
- (2) Pleitez, M. von Lilienfeld-Toal, H. Mäntele W. Infrared spectroscopic analysis of human interstitial fluid in vitro and in vivo using FT-IR spectroscopy and pulsed quantum cascade lasers (QCL): Establishing a new approach to non invasive glucose measurement Spectrochimica acta. Part A, Molecular and biomolecular spectroscopy, 85:61-65, 2012
- (3) Pleitez, M. et al. In Vivo Noninvasive Monitoring of Glucose Concentration in Human Epidermis by Mid-Infrared Pulsed Photoacoustic Spectroscopy Analytical Chemsitry, 85:1013-1020, 2013.
- (4) Pleitez, M. Lieblein, T. Bauer, A. Hertzberg, o. von Lilienfeld-Toal , H. Mäntele, W. Windowless ultrasound photoacoustic cell for in vivo mid-IR spectroscopy of human epidermis: Low interference by changes of air pressure, temperature, and humidity caused by skin contact opens the possibility for a non-invasive monitoring of glucose in the interstitial fluid Review ofScientific Instruments 84, 2013
- (5) M. A. Pleitez Rafael, o. Hertzberg, A. Bauer, M. Seeger, T. Lieblein, H. von Lilienfeld-Toal , and W. Mäntele. Photo-thermal deflectometry enhanced by total internal reflection enables non- invasive glucose monitoring in human epidermis. The Analyst, November 2014.

Aus der DE 31 46 700 ist ein Verfahren der Infrarotspektroskopie bekannt, bei dem mit monochromatischem Licht eines Anregungsstrahls eine Probe angeregt und die infolge von Absorptionseffekten thermisch induzierten Dichteschwankungen optisch mit Hilfe eines zweiten Lichtstrahls nachgewiesen werden. Der zweite Lichtstrahl nutzt eine aus der ATR-Spektroskopie bekannte Platte als optisches Medium, deren Brechungsindex durch die Wärmeeinwirkung lokal geändert wird. Die Brechzahländerungen in der ATR-Platte finden somit mit der Modulationsfrequenz des Anregungslichts statt, so dass aus der Amplitude der Brechzahländerung, nachgewiesen durch eine Amplitude der Intensität des Messlichts nach Durchlaufen der ATR-Platte, auf die Dichte einer nachzuweisenden Substanz in der Probe geschlossen werden kann.

Die US 2013/0286397 bezieht sich auf ein Array von einstellbaren Quantenkaskadenlasern. Hiermit verbunden ist ein Linsenarray, bei dem einzelne Linsen den einzelnen Lasern des Arrays zugeordnet sind. Die Schrift erwähnt die Anwendung zur Glukosemessung entweder als Reflexions- oder Absorptionsmessung. Es ist zudem erwähnt, dass Laserarray und Linsenarray in einem Block kombiniert werden können, um eventuelle thermische Ausdehnungen in beiden Arrays auszugleichen.

Die EP 1 048 265 A1 beschreibt eine Messmethode für die Glukosespiegelbestimmung, die spektroskopisch im mittleren Infrarotspektralbereich arbeitet und die Absorption eines Anregungslichtstrahls durch das nachzuweisende Medium durch akustische Impulse nachweist, die durch die entstehenden Temperaturgradienten erzeugt werden. Hierzu werden zumindest zwei verschiedene Wellenlängen eingestrahlt, von denen eine durch Glukose absorbiert wird und die andere der Kalibrierung dient. Die Anwendung der Methode im nahen Infrarotgebiet wird abgelehnt.

Aus der DE 44 46 390 ist ein Verfahren bekannt, bei dem im Infrarotbereich zwei Strahlen durch Laserdioden erzeugt werden, wobei beide Strahlen mit derselben Frequenz intensitätsmoduliert und um 180° gegeneinander phasenverschoben sind. Durch Abgleich der Modulationsamplituden der Strahlen, die unterschiedliche Wellenlängen aufweisen, kann erreicht werden, dass eine Gesamtstrahlungsintensität zeitlich konstant ist, solange der aus den beiden Teilstrahlen zusammengesetzte Lichtstrahl keine Probe durchläuft. Wird eine Probe durchlaufen, so können die Amplituden so weit ausgeglichen werden, dass die gleichbleibende Intensität wieder hergestellt ist. Die hierzu notwendige Intensitätsänderung einer der Lichtquellen gibt Aufschluss über die Konzentration eines Stoffs in der durchstrahlten Probe, die wenigstens einen der Lichtstrahlen absorbiert.

Die DE 10 2005 048 807 offenbart ein Infrarotanalyse-Verfahren von Inhaltsstoffen einer Probe, die sich eines ATR-Körpers als optischen Mediums bedient, das mit der zu vermessenden Flüssigkeit in Kontakt steht. Das eingestrahlte Licht kann aus Komponenten mit unterschiedlichen Wellenlängen zusammengesetzt werden. Die unterschiedlichen Lichtstrahlen mit verschiedenen Wellenlängen können durch verschiedene Modulationsfrequenzen und Pulsmuster sowie verschiedene Intensitäten codiert werden. Die Summe aller Lichtsignale wird durch den ATR-Körper transportiert und dort durch ein- oder mehrfache Reflexion mit dem der zu vermessenden Probe in Wechselwirkung gebracht. Der ATR-Körper ist vorzugsweise als planparallele Platte ausgebildet und lässt somit in einfacher Weise Multireflexionen zu. Als Lichtquelle des Anregungslichts sind Quantenkaskadenlaser sowie Arrays aus Quantenkaskadenlasern erwähnt. Pulsdauern und Intensitäten des Anregungslichts können in Abhängigkeit von dem nachzuweisenden Stoff gestaltet werden. Es können auch unterschiedliche Wellenlängen gleichzeitig abgestrahlt werden, womit komplexe zu untersuchende Zusammensetzungen der Probe vermessen werden können.

Es liegt die Aufgabe zugrunde, eine Vorrichtung anzugeben, mit der sich ein Stoff, insbesondere ein tierisches oder menschliches Gewebe oder ein Bestandteil oder Inhaltsstoff des Gewebes, besonders einfach und kostengünstig analysieren lässt.

Diese Aufgabe wird unter anderem durch eine Vorrichtung mit den Merkmalen gemäß Anspruch 1 gelöst. Ausgestaltungen der Vorrichtung sind in Unteransprüchen angegeben. Verwiesen wird auf die deutsche Patentschrift DE 10 2014 108 424 B3, die eine Vorrichtung nach dem Oberbegriff des Anspruchs 1 offenbart, auf deren Inhalt hier konkret Bezug genommen wird und auf die diese Anmeldung inhaltlich aufbaut. Insbesondere bezieht sich diese Bezugnahme auf sämtliche in den erteilten Patentansprüchen genannten Merkmale. Darüber hinaus bezieht sich die Bezugnahme insbesondere auch auf Details des dort genannten Anregungs-Lichtstrahls, beispielsweise auf die dort genannten Zahlenwerte der Pulsfrequenzen und Wellenlängen(-bereiche), und ebenso auf die Details zur Messung von Glukosegehalt in der interstitiellen Flüssigkeit.

Die vorliegende PCT-Schutzrechtsanmeldung bezieht sich neben den unmittelbar bei Einreichung ausdrücklich genannten Anspruchsgegenständen und Ausführungsbeispielen auch auf weitere Aspekte, die am Ende der hiesigen Beschreibung aufgezählt sind. Diese Aspekte können einzeln oder in Gruppen jeweils mit Merkmalen der bei Einreichung genannten Ansprüche kombiniert werden. Diese Aspekte stellen für sich genommen, oder miteinander, oder mit Anspruchsgegenständen kombiniert, eigenständige Erfindungen dar. Die Anmelderin behält sich vor, diese Erfindungen zu einem späteren Zeitpunkt zum Gegenstand von Ansprüchen zu machen. Dies kann im Rahmen dieser Anmeldung oder auch im Rahmen von späteren Teilanmeldungen, Fortsetzungsanmeldungen ("continuation applications" in den USA), Teil-Fortsetzungsanmeldungen ("continuation-in-part applications" in den USA) oder Nachanmeldungen unter Inanspruchnahme der Priorität dieser Anmeldung geschehen.

Im Folgenden wird aber zunächst auf die bei Einreichung aufgeführten Anspruchsgegenstände eingegangen.

Es ist eine Vorrichtung zum Analysieren eines Stoffs vorgesehen mit einer Anregungssendeeinrichtung zum Erzeugen mindestens eines elektromagnetischen Anregungsstrahls, insbesondere Anregungs-Lichtstrahls, mit zumindest einer Anregungswellenlänge, einer Detektionseinrichtung zur Detektion eines Reaktionssignals und einer Einrichtung zum Analysieren des Stoffes anhand des detektierten Reaktionssignals.

Ein wesentlicher Vorteil dieser Vorrichtung ist darin zu sehen, dass sich mit dieser sehr einfach und zuverlässig ein Stoff analysieren lässt.

Unter dem Begriff Licht werden hier elektromagnetische Wellen bzw. elektromagnetische Strahlung im sichtbaren Bereich, im nahen und fernen Infrarotbereich sowie um UV-Bereich verstanden.

Bei einer beispielhaften Ausgestaltung der Vorrichtung ist vorgesehen, dass
die Anregungssendeeinrichtung eine Strahlungsquelle, in einer Ausführungsform eine monochromatische, insbesondere eine polarisierte Strahlungsquelle oder Lichtquelle, weiter insbesondere eine Laserlichtquelle ist,
- die Vorrichtung ein optisches Medium aufweist, das mit dem Stoff, insbesondere einem ersten Bereich der Oberfläche des Stoffs, in direktem Kontakt steht,
- wobei vorzugsweise die Anregungssendeeinrichtung so angeordnet ist, dass der ausgesendete Anregungsstrahl in das optische Medium eindringt und dieses an einem vorbestimmten Punkt an der Oberfläche des optischen Mediums wieder verlässt, und
- die Vorrichtung eine Einrichtung zum Aussenden eines Messstrahls, insbesondere Mess-Lichtstrahls, umfasst, die so angeordnet ist, dass der ausgesendete Messstrahl in das optische Medium eindringt und wobei vorzugsweise im Betrieb der Messstrahl und der Anregungsstrahl sich auf einer Grenzfläche des optischen Mediums und der Oberfläche des Stoffs, an der der Messstrahl reflektiert wird, überlappen, und
- die Detektionseinrichtung eine Einrichtung zum Empfangen des das Reaktionssignal bildenden reflektierten Messstrahls und/oder zum direkten oder indirekten Detektieren einer Ablenkung des reflektierten Messstrahls ist.

Vorzugsweise weist die Vorrichtung ein optisches Medium auf, das mit dem Stoff, insbesondere einem ersten Bereich der Oberfläche des Stoffs, in einer Ausführungsform der Haut eines Menschen, in direktem Kontakt steht wobei die Detektionseinrichtung zur Detektion eines Reaktionssignals eine Parameter-änderung des optischen Mediums, insbesondere in einem dem ersten Bereich benachbarten Bereich, infolge des Reaktionssignals, insbesondere eine Verformung und/oder Dichteänderung des optischen Mediums, als Folge einer lokalen, zeitabhängigen Erwärmung, erfasst. Das optische Medium kann aus einem optisch oder für Infrarotstrahlung oder Ultraviolettstrahlung, allgemein für den Anregungsstrahl und den Messstrahl transparenten Material, beispielweise Glas, Kristall, Zinksulfid (ZnS), Zinkselenid (ZnSe), Germanium (Ge), Silizium (Si) und Diamant oder einem transparenten Kunststoff, in einer Ausführungsform einem Polyethylen, bestehen. Eine lokale Erwärmung als Reaktion auf einen Wärmetransport von dem zu analysierenden Stoff oder einer Substanz des Stoffes in das optische Medium führt dort zu einer Veränderung, beispielsweise einer Materialverformung oder zu thermischen Spannungen oder lokalen Veränderungen im Brechungsindex, die detektierbar sind.

Der Stoff kann dabei in einer Ausführungsform das Gewebe eines Lebewesens insbesondere eines Menschen sein, wobei die Stoffoberfläche die Haut sein kann. Es können dann Substanzen in dem Gewebe analysiert oder gemessen werden.

Es kann zudem vorgesehen sein, dass die Detektionseinrichtung einen mit dem optischen Medium verbundenes oder in dieses integriertes Piezoelement als Detektor zur Erfassung einer Spannung, Verformung und/oder Dichteänderung aufweist.

Außerdem kann vorgesehen sein, dass die Detektionseinrichtung wenigstens einen Temperatursensor als Detektor zur Erfassung des Reaktionssignals aufweist. Dieser kann direkt am optischen Medium angeordnet sein oder in seiner Umgebung, in Abhängigkeit vom Messprinzip.

Vorzugsweise weist die Vorrichtung eine Einrichtung zur Intensitätsmodulierung des Anregungslichtstrahls auf.

Die Detektionseinrichtung ist bevorzugt zur Detektion eines zeitabhängigen Reaktionssignals in Abhängigkeit von der Wellenlänge des Anregungslichts und/oder der Intensitäts-Modulation des Anregungslichts geeignet.

Auch kann es vorgesehen sein, dass die Anregungssendeeinrichtung mindestens einen elektromagnetischen Anregungsstrahl in ein Stoffvolumen einstrahlt, das unterhalb eines ersten Bereiches der Oberfläche des Stoffs liegt.

Besonders bevorzugt umfasst die Anregungssendeeinrichtung zwei oder mehr Sendeelemente, insbesondere in Form eines ein- zwei- oder mehrdimensionalen Sendeelementarrays. Dies kann somit als Flächenarray von Sendeelementen oder auch als Sendeelementleiste ausgebildet sein (in einer Ausführungsform Halbleiterlaserarrays oder QCL-Arrays, wobei QCL für Quantenkaskadenlaser steht).

Zudem kann vorgesehen sein, dass die zwei oder mehr Sendeelemente jeweils einen eigenen elektromagnetischen Anregungsstrahl erzeugen und diesen in das Volumen unterhalb des ersten Bereiches einstrahlen. Die verschiedenen Anregungsstrahlen können nacheinander oder auch wenigstens teilweise gleichzeitig ausgesendet werden. Die verschiedenen Sendeelemente können auch zeitgleich mit verschiedenen Modulationsfrequenzen betrieben werden.

Die Wellenlängen der elektromagnetischen Anregungsstrahlen der zwei oder mehr Sendeelemente unterscheiden sich vorzugsweise. Die Wellenlängen sind bevorzugt derart gewählt, dass eine in dem zu analysierenden Stoff zu detektierende Substanz Strahlung dieser Wellenlängen besonders gut absorbiert. Es können auch zusätzlich oder alternativ Wellenlängen oder Wellenlängenbereiche gewählt werden, die die zu detektierende Substanz nicht absorbiert, die aber von anderen Substanzen absorbiert werden (sogenannte tolerante Wellenlängen), um die zu analysierende Substanz von anderen Substanzen abgrenzen zu können. Die Anregungssendeeinrichtung umfasst in einer Ausführungsform zwei oder mehr Laser, insbesondere in Form eines ein- oder zweidimensionalen Laserarrays, wobei auch mehrere Reihen von Laserelementen zur Platzersparnis gestaffelt und versetzt hintereinander angeordnet sein können, in einer Ausführungsform einer Laserleiste, und/oder zwei oder mehr Leuchtdioden, insbesondere in Form eines ein- oder zweidimensionalen Diodenarrays, auch tiefengestaffelt und gegeneinander versetzt, in einer Ausführungsform eines flächigen Arrays oder einer Leiste. Die Outputstrahlen der Arrays können sowohl, nahe beisammen oder parallel, für jedes Strahlelement einzeln, als auch durch bereits integrierte Optiken, denselben Strahlengang haben.

Bezüglich des Aufbaus der Vorrichtung kann es vorgesehen sein, dass die Anregungssendeeinrichtung unmittelbar oder mittelbar - vorzugsweise mittels einer Justagevorrichtung - mit einem optischen Medium, das mit dem Stoff, insbesondere dem ersten Bereich der Oberfläche des Stoffs, in direktem Kontakt steht, mechanisch fest verbunden ist. Somit kann die Anregungssendeeinrichtung bereits bei der Herstellung oder zumindest vor dem Einsatz im Betrieb relativ zu dem optischen Medium justiert und fixiert werden.

Das optische Medium kann zur Befestigung und/oder Ausrichtung oder Justage von einer Anregungssendeeinrichtung und/oder Elementen einer Detektionseinrichtung wenigstens eine integrierte Erhebung und/oder Ausnehmung, wie einen Steg, eine Schulter, eine aufgesetzte Halbkugel, einen aufgesetzten Quader, einen Kegel oder eine Bohrung, Nut, Mulde oder sonstige Ausnehmung aufweisen, in oder an die die genannten Elemente (die Anregungssendeeinrichtung und /oder Elemente einer Detektionseinrichtung) angelegt, angelehnt oder an denen sie justiert oder befestigt werden können. Auch das Anarbeiten von ausgerichteten Passflächen durch Bearbeiten des optischen Mediums oder in einem Gießprozess kann vorgesehen sein.

Bezüglich der Einrichtung zur Intensitätsmodulierung kann vorgesehen sein, dass diese eine elektrische oder elektromechanische Modulationseinrichtung, die mit der Anregungssendeeinrichtung elektrisch in Verbindung steht und diese insbesondere elektrisch ansteuert, umfasst oder durch eine solche gebildet ist. Die Modulationseinrichtung kann eine Intensitätsmodulierung des Anregungsstrahls, in einer Ausführungsform eine periodische Intensitätsmodulierung, weiter beispielsweise in Form von Rechteckpulsen, einer Sägezahnfunktion oder einer Sinusfunktion oder einer anderen periodischen Funktion erzeugen.

Alternativ oder zusätzlich kann die Einrichtung zur Intensitätsmodulierung wenigstens einen im Strahlengang angeordneten gesteuerten Spiegel umfassen, durch dessen Ansteuerung die Intensität des Anregungsstrahls durch Ablenkung modulierbar ist.

Alternativ oder zusätzlich kann die Einrichtung zur Intensitätsmodulierung wenigstens eine bezüglich ihrer Transparenz steuerbare, im Strahlengang angeordnete Schicht umfassen oder durch eine solche gebildet sein. Somit kann das Modulationselement in Form eines bezüglich seiner Transmission gesteuerten Transmissionselementes ausgestaltet sein. Das Modulationselement kann aus einem Lichtstrahl mehrere, räumlich getrennte Lichtstrahlen erzeugen. Es kann in einer Ausführungsform auch vorgesehen sein, dass mit dem Modulationselement die Oberfläche einer Probe abgerastert wird. Das Modulationselement kann in einer Ausführungsform gemeinsam mit dem Array von Lichtquellen/Laserquellen gesteuert werden.

Eine Einrichtung zum Aussenden eines Messstrahls, insbesondere Mess-Lichtstrahls, ist in einer Ausführungsform zum Aussenden des Messstrahls in denjenigen Bereich eines optischen Mediums vorgesehen, der mit dem ersten Bereich der Oberfläche des Stoffs in Kontakt steht.

Die Einrichtung zum Aussenden eines Messstrahls und die Detektionseinrichtung sind in einer Ausführungsform derart zueinander ausgerichtet, dass die Detektionseinrichtung als das zeitabhängige Reaktionssignal den Messstrahl detektiert, nachdem dieser zumindest einmal an derjenigen Grenzfläche des optischen Mediums reflektiert worden ist, die mit dem Stoff, insbesondere dem ersten Bereich der Oberfläche des Stoffs, in Kontakt steht.

Mit Blick auf eine einfache Montage ist es vorteilhaft, wenn die Einrichtung zum Aussenden eines Messstrahls und/oder die Detektionseinrichtung und/oder die Anregungssendeeinrichtung unmittelbar mit dem optischen Medium mechanisch fest verbunden und/oder mittels eines oder mehrerer Lichtwellenleiter an dieses angekoppelt ist.

Auch sind Ausgestaltungen denkbar, bei denen das optische Medium unmittelbar eine Abbildungsoptik trägt und/oder in das optische Medium eine Abbildungs-Optik integriert ist.

Darüber hinaus sind Ausgestaltungen denkbar, bei denen die Oberfläche des optischen Mediums mehrere gegeneinander geneigte Teilflächen aufweist, an denen der Messstrahl, insbesondere der Mess-Lichtstrahl, mehrfach reflektiert wird.

Auch können Ausgestaltungen vorgesehen sein, bei denen in oder an dem optischen Medium ein oder mehrere Spiegelflächen zur Reflektion des Messstrahls, insbesondere Mess-Lichtstrahls, vorgesehen sind.

Mit Blick auf einen kompakten Aufbau ist es denkbar, dass die Anregungssendeeinrichtung und/oder die Einrichtung zum Aussenden des Messstrahls und/oder die Detektionseinrichtung unmittelbar aneinander oder an einem gemeinsamen Träger befestigt sind. Die verschiedenen Einrichtungen können an dem Träger in einer Ausführungsform durch Schweißen oder Kleben oder durch Verschrauben oder durch eine Rastverbindung befestigt sein, wobei eine Justagemöglichkeit entweder beim Zusammenbau oder auch später durch eine Justierschraube oder eine andere mechanische Justiereinrichtung gegeben sein kann. Insbesondere die Einrichtung zum Aussenden des Messstrahls und/oder die Detektionseinrichtung sollten gut zueinander justiert oder justierbar sein. Darum kann es sinnvoll sein, diese beiden Einrichtungen unmittelbar an dem optischen Medium zu befestigen. Die Einrichtung zum Aussenden des Messstrahls und/oder die Detektionseinrichtung können bei geeigneter Lenkung des Messstrahls auch auf derselben Seite des optischen Mediums nebeneinander angeordnet und auf einem gemeinsamen Träger, in einer Ausführungsform einer gemeinsamen Leiterplatte oder einem gemeinsamen Halbleiter befestigt oder als gemeinsame integrierte Halbleitereinrichtung, in einer Ausführungsform als gemeinsamer integrierter Halbleiterbaustein ausgebildet sein. Dieser Träger kann dann als Ganzes gegenüber dem optischen Medium justiert werden, in einer besonderen Ausführungsform auch ohne die Relativposition zwischen der Einrichtung zum Aussenden des Messstrahls und/oder der Detektionseinrichtung noch zu verändern.

Der Träger ist bevorzugt durch eine Leiterplatte, eine Metallplatte oder Kunststoffplatte oder ein Gehäuse oder Gehäuseteil der Vorrichtung gebildet.

Es kann außerdem vorgesehen sein, dass die Anregungssendeeinrichtung einen integrierten Halbleiterbaustein umfasst, der einen oder mehrere Laserelemente sowie mindestens ein mikrooptisches Bauelement und vorzugsweise zusätzlich ein Modulationselement aufweist. Die genannten Elemente können gemeinsam aus einem Halbleiterrohling hergestellt, in einer Ausführungsform geätzt sein oder zumindest in einem gemeinsamen Gehäuse untergebracht sein.

Es kann zudem vorgesehen sein, dass das Modulationselement wenigstens ein gegenüber dem übrigen Halbleiterbaustein bewegliches und bezüglich einer Position steuerbares Element, insbesondere einen Spiegel aufweist. Dieser kann durch eine MEMS-Einrichtung ansteuerbar sein.

Es kann auch vorgesehen sein, dass das Modulationselement eine in ihrer Strahlungsdurchlässigkeit steuerbare Schicht aufweist.

Es kann auch vorgesehen sein, dass das Modulationselement eine elektronische Ansteuerschaltung für die Modulation der einen oder mehreren Laserelemente aufweist. Das Modulationselement kann in einer Ausführungsform derart beschaffen sein, dass es durch Interferenz, Phasenversatz/Gangversatz oder eine Polfiltereinrichtung oder andere bekannte Modulationsmechanismen den Anregungsstrahl zeitabhängig verändert.

Das oder die mikrooptischen Bauelemente können als in das Halbleiterbauelement integrierte oder aus diesem herausgearbeitete, insbesondere durch Ätzen herausgearbeitete Spiegel oder Linsen ausgeführt sein.

Die beschriebene Vorrichtung zum Analysieren eines Stoffes kann einen Messwert für eine Stoffkonzentration, in einer Ausführungsform eine Glucosekonzentration ermitteln. Die Vorrichtung kann eine Schnittstelle zu einer Anzeigeeinrichtung von Messwerten und ihrer Bewertung, beispielsweise durch einen Farbcode, für einen Benutzer der Vorrichtung und/oder zu einer Dosiereinrichtung für eine Substanz aufweisen, die in den Stoff, insbesondere das Gewebe oder allgemein den Körper eines Lebewesens abgegeben werden kann. Die Vorrichtung kann eine solche Dosiervorrichtung auch unmittelbar mit umfassen. Dabei kann die Vorrichtung zudem eine Einrichtung zur Erfassung oder Analyse der Stoffoberfläche, in einer Ausführungsform Hauoberfläche oder in einer Ausführungsform auch Augenfläche oder Iris eines Lebewesens aufweisen, die eine Identifizierung einer Person oder eines Lebewesens aufgrund eines Vergleiches mit Referenzdaten ermöglicht und somit dazu dienen kann, sicherzustellen, dass geeignete Referenzwerte und /oder Kalibrierungswerte für die Analyse des Stoffes und die Steuerung der Dosiervorrichtung bereitgestellt werden. Ermittelte Kennwerte der Stoffoberfläche, in einer Ausführungsform ein Fingerabdruck oder die Struktur einer Iris eines Auges, können außer zur Identifizierung und Authentifizierung einer Person, auch gegenüber einer Datenbank, ebenso zur Verschlüsselung der Kommunikation von Zustandswerten und der Steuerung der Dosiereinrichtung, die, verschlüsselt oder unverschlüsselt, grundsätzlich von der Datenbank aus geschehen kann, dienen. Die Dosiereinrichtung kann in einer Ausführungsform mit einem Sensor zur Ermittlung eines Füllstandes einer abzugebenden Substanz, wie in einer Ausführungsform Insulin und/oder Glucagon versehen sein und eine Einrichtung aufweisen, um den Füllstand zu der Vorrichtung zur Stoffanalyse und/oder direkt an die Datenbank zu übermitteln.

Zudem kann die Vorrichtung eine Schnittstelle, in einer Ausführungsform eine Funkschnittstelle zu der Datenbank aufweisen, zu der die Messwerte geschickt werden können und die die Daten weiterverarbeiten kann. Die Datenbank kann derart beschaffen sein, dass sie die Daten mehrerer Patienten , das heißt in einer Ausführungsform auch die Daten von mehreren gleichartigen Vorrichtungen zum Analysieren eines Stoffes verarbeitet und speichert und in einer Ausführungsform auch individuelle Dosiereinrichtungen zur Abgabe von Substanzen steuert. Die Datenbank kann auch die ermittelten Daten über den analysierten Stoff weiter verarbeiten und abgeleitete Analyseergebnisse wie einen Trend der werte, zeitliche erste und zweite Ableitungen, Minima, Maxima, Standardabweichungen von Stoffmengen oder -Konzentrationen, Blutzuckerwerten oder anderen physiologischen Werten von Patienten ermitteln, vergleichen und daraus Signale, in einer Ausführungsform auch Alarmsignale ableiten. Auch der Füllstand der Dosiereinrichtung kann von der Datenbank erfasst und verarbeitet werde, um in einer Ausführungsform eine zeitliche Reichweite der Füllung oder die Notwendigkeit einer Nachfüllung zu ermitteln und direkt an die Vorrichtung des Patienten oder eine Serviceeinrichtung zu signalisieren. Hierzu kann die Datenbank mit einer Kommunikationseinrichtung in einer Serviceeinrichtung, in einer Ausführungsform einem Krankenhaus oder einer Arztpraxis verbunden sein. Zum Zweck der Übermittlung von Daten von und/oder zu einer Datenbank kann die Vorrichtung in einer Ausführungsform mittels einer Funkverbindung, in einer Ausführungsform Bluetooth oder WLan oder Wifi oder anderer Übertragungsmethoden mit einem Mobilfunkgerät oder einem Pager verbunden sein. Die Vorrichtung kann auch unmittelbar mit einer WLAN- Schnittstelle und einem Internet-Client versehen sein.

Der Gegenstand bezieht sich außerdem auf ein Verfahren zum Analysieren eines Stoffes, wobei bei dem Verfahren mit einer Anregungssendeeinrichtung mindestens ein elektromagnetischer Anregungsstrahl, mit zumindest einer Anregungswellenlänge durch den wenigstens teilweise gleichzeitigen oder aufeinander folgenden Betrieb mehrerer Laseremitter einer Laserlichtquelle erzeugt wird, mit einer Detektionseinrichtung ein Reaktionssignal detektiert wird und anhand des detektierten Reaktionssignals der Stoff analysiert wird. Dabei kann die thermische Diffusivität in dem Stoff und der zeitliche Verlauf des Reaktionssignals zur Charakterisierung der Natur des Stoffes oder einer örtlichen Verteilung einer Substanz in dem Stoff dienen oder zur Charakterisierung der Tiefe, in der der Anregungsstrahl absorbiert wird.

Es kann in einer Ausführungsform vorgesehen sein, dass nacheinander unter Verwendung verschiedener Modulationsfrequenzen der Anregungssendeeinrichtung Reaktionssignale, insbesondere zeitliche Reaktionssignalverläufe, ermittelt werden und dass mehrere Reaktionssignalverläufe zu verschiedenen Modulationsfrequenzen miteinander verknüpft werden und dass daraus insbesondere eine für einen Tiefenbereich unter der Stoffoberfläche spezifische Information gewonnen wird.

Es kann auch vorgesehen sein, dass Reaktionssignalverläufe zu verschiedenen Modulationsfrequenzen jeweils für verschiedene Wellenlängen des Anregungsstrahls ermittelt werden und daraus insbesondere jeweils eine für einen Tiefenbereich unter der Stoffoberfläche spezifische Information gewonnen wird. Bei der Verwendung von mehreren Modulationsfrequenzen des Pumpstrahls zur gleichen Zeit ist es beispielsweise möglich das detektierte Signal mittels eines entsprechenden Analyseverfahren, beispielsweise Fourier-Transformation, ihren Frequenzen entsprechend aufzulösen; die FT würde nur das Signal herausfiltern das der gewünschten Frequenz entspricht.

Es kann auch vorgesehen sein, dass ein optisches Medium mit dem Stoff, insbesondere einem ersten Bereich der Oberfläche des Stoffs, in direkten Kontakt gebracht wird, mit der Anregungssendeeinrichtung der ausgesendete Anregungsstrahl erzeugt und insbesondere derart ausgestrahlt wird, dass dieser in das optische Medium eindringt und dieses an einem vorbestimmten Punkt an der Oberfläche des optischen Mediums wieder verlässt, mit einer Einrichtung zum Aussenden eines Messstrahls ein Messstrahl, insbesondere Mess-Lichtstrahl, derart erzeugt wird, dass dieser in das optische Medium eindringt und dass insbesondere der Messstrahl und der Anregungsstrahl sich im Betrieb auf einer Grenzfläche des optischen Mediums und der Oberfläche des Stoffs, an der der Messstrahl reflektiert wird, überlappen, und mit der Detektionseinrichtung ein das Reaktionssignal bildender reflektierter Messstrahl gemessen und/oder direkt oder indirekt die Ablenkung des reflektierten Messstrahls detektiert wird.

Ein Aspekt des Verfahrens ist die Fokussierung der Messung des Reaktionssignals auf selektierte Tiefenbereiche unterhalb der (Abstandsintervalle von der) Stoffoberfläche. Die thermische Wellenlänge d hat dabei den größten Einfluss auf den mit dem Verfahren gemessenen Tiefenbereich. Sie ist definiert als d = √(D/(π*f)). Wobei D die thermische Diffusivität der Probe (hier bspw. Haut) ist und f die Modulationsfrequenz des Anregungstrahls. Literatur zur thermischen Diffusivität von Haut:
- U. Werner, K. Giese, B. Sennhenn, K. Plamann, and K. Kölmel, "Measurement of the thermal diffusivity of human epidermis by studying thermal wave propagation," Phys. Med. Biol. 37(1), 21-35 (1992).
- A. M. Stoll, Heat Transfer in Biotechnology, Vol 4 of Advances in Heat Transfer, J. P. Hartnett and T. Irvin, eds. (New York, Academic, 1967), p 117.

Zur Eliminierung von Reaktionssignalen aus den obersten Schichten des Stoffes können in einer Ausführungsform Änderungen der Messwerte im Vergleich zu vorangegangenen Messungen herangezogen werden, falls die Messwerte in den obersten Schichten sich im Vergleich zu anderen, tieferliegenden Schichten weniger oder langsamer verändern. Dies kann in einer Ausführungsform bei Messungen an der menschlichen Haut der Fall sein, wo die obersten Hautschichten praktisch keinem Austausch mit den unteren Schichten unterliegen und deshalb physiologische Parameter wenig veränderlich sind. Es kann auch die zeitliche Ableitung von Messwerten zu Reaktionssignalen zum Ausschluss der Signale aus den obersten Hautschichten herangezogen werden. So kann die Messung oder zumindest die Auswertung auf die interstitielle Flüssigkeit in der Haut begrenzt oder fokussiert werden.

Außerdem kann vorgesehen sein, dass in Abhängigkeit von einer in dem Stoff ermittelten Stoffkonzentration eine Dosiereinrichtung zur Abgabe einer Substanz, insbesondere in einen Patientenkörper angesteuert wird und/oder ein akustisches und/oder optisches Signal ausgegeben wird und/oder ein Signal mittels einer Funkverbindung an eine Verarbeitungseinrichtung abgegeben wird. Dabei können außer einem aktuell ermittelten Messwert auch eine zeitliche Messwertentwicklung, eine Ableitung des Messwertes, Durchschnittwerte von Messwerten, Maxima, Minima, eine Standardabweichung sowie vorgegebene Schwellen für Messwerte berücksichtigt und mit dem aktuellen Messwert verknüpft werden. Die Verarbeitungseinrichtung kann in einer Ausführungsform eine Datenbank sein oder mit einer Datenbank verbunden sein, die Daten mehrerer Patienten sammelt und verarbeitet. Die Datenbank kann direkt mit einer Steuerungseinrichtung der Vorrichtung verbunden oder entfernt von dieser und über eine Kommunikationsschnittstelle angebunden sein.

Um eine erhöhte Sicherheit beim Betrieb einer Dosiereinrichtung, insbesondere für Insulin, zu erreichen, kann vorgesehen sein, dass diese mittels eines voreingestellten Standardverfahrens mit vorgewählten Mengenabgaben zu bestimmten oder bestimmbaren Zeiten lokal oder von einer Datenbank aus gesteuert betrieben wird und dass mittels der oben beschriebenen Vorrichtung sinnvolle Abweichungen von voreingestellten Abgabewerten ermittelt werden, die zu einer Korrektur und Verbesserung der Ansteuerung der Dosiereinrichtung verwendet werden. Auf diese Weise ist auch bei einem Ausfall der Vorrichtung zumindest ein Normal- oder Notbetrieb der Dosiereinrichtung gewährleistet

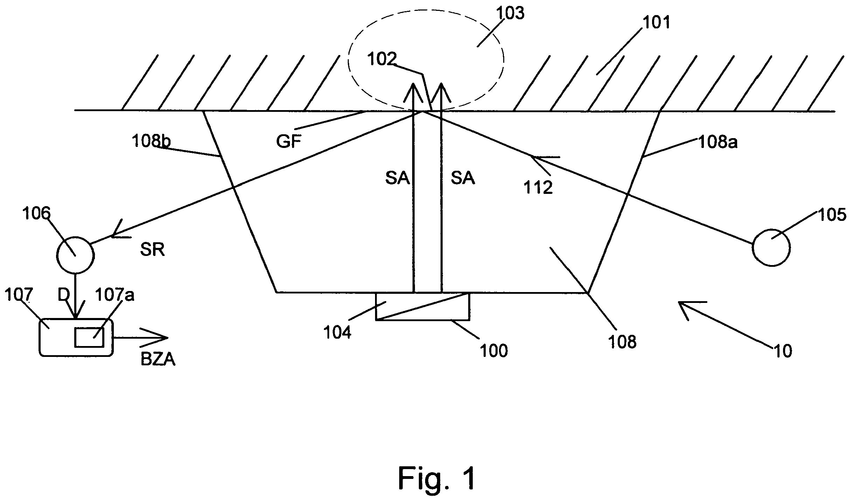

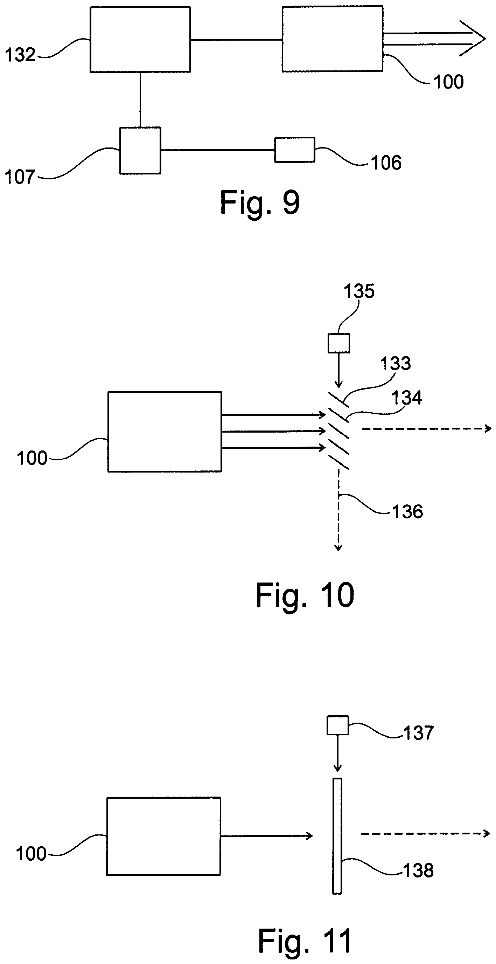

Die Figuren 1 bis 13 zeigen schematisch verschiedene Elemente der Vorrichtung und ihrer Elemente teilweise in unterschiedlichen Ausführungsformen.

Die Figur 1 zeigt ein Ausführungsbeispiel für eine Vorrichtung 10 zum Analysieren eines Stoffes 101. Der Stoff 101 liegt vorzugsweise unmittelbar auf einem optischen Medium 108 auf, das als optisch transparenter Kristall oder Glaskörper ausgebildet sein kann. Die Vorrichtung zum Analysieren des Stoffes 101 dient zum Beispiel zum Messen des Glukose- bzw. Blutzuckergehalts in einer Flüssigkeit, wie in einer Ausführungsform Blut, und zur Erzeugung einer Glukose- bzw. Blutzuckerspiegelangabe BZA.

Die Vorrichtung umfasst eine Anregungssendeeinrichtung 100 zum Aussenden eines oder mehrerer elektromagnetischer Anregungsstrahlen SA, vorzugsweise in Form von Anregungs-Lichtstrahlen mit einer oder mehreren Anregungswellenlängen, in ein Volumen 103, das in dem Stoff 101 unterhalb eines ersten Bereiches 102 der Oberfläche des Stoffes liegt. Die Anregungssendeeinrichtung 100 wird nachfolgend auch kurz als Anregungs-Lichtquelle 100 bezeichnet. Bei der Anregungs-Lichtquelle 100 kann es sich um einen bezüglich der Wellenlänge durchstimmbaren Laser, insbesondere durchstimmbaren Quantenkaskadenlaser, handeln; bevorzugt wird, wie weiter unten noch erläutert, eine Lichtquellenleiste oder ein Lichtquellenarray mit zumindest zwei Einzelemittern, insbesondere Halbleiterlasern, eingesetzt, mit denen jeweils eine vorgegebene individuelle Wellenlänge emittiert wird.

Außerdem ist eine Einrichtung 104 zur Intensitätsmodulierung des oder der Anregungslichtstrahlen SA vorgesehen, die vorzugsweise durch eine Modulationseinrichtung für die Anregungs-Lichtquelle, insbesondere ihrer Ansteuerung, und/oder wenigstens einen im Strahlengang angeordneten gesteuerten Spiegel und/oder eine bzgl. ihrer Transparenz steuerbare und im Strahlengang angeordnete Schicht gebildet ist.

Zudem weist die Vorrichtung eine Einrichtung 105 zum Aussenden eines elektromagnetischen Messstrahls 112, insbesondere Mess-Lichtstrahls, auf, der an der Grenzfläche GF zwischen dem Stoff 101 und dem optischen Medium 108 reflektiert, vorzugsweise totalreflektiert, wird.

Eine Detektionseinrichtung 106 dient zur Detektion des reflektierten Messstrahls 112, der ein zeitabhängiges Reaktionssignal SR bildet; die Amplitude des Reaktionssignals SR wird von der Wellenlänge des Anregungslichts SA und der Intensitäts-Modulation des Anregungslichts SA beeinflusst, wie weiter unten anhand von Beispielen noch näher erläutert wird.
Die Amplitude des Messsignals hängt hierbei von der Wellenlänge des Anregestrahls, den Absorptionseigenschaften der Probe, sowie den thermischen Eigenschaften, insbesondere der thermischen Diffusivität und thermischen Leitfähigkeit der Probe als auch des optischen Elementes ab. Darüber hinaus spielt auch die Kopplung des thermischen Signals von der Probe in das optische Element hinein eine Rolle.

Eine Einrichtung 107 zum Analysieren des Stoffes wertet die detektierten Reaktionssignale SR aus und erzeugt in einer Ausführungsform eine Glukose- bzw. Blutzuckerspiegelangabe BZA.

Nachfolgend soll der Betrieb der Vorrichtung 10 gemäß Figur 1 und in diesem Zusammenhang ein Verfahren zum Analysieren eines Stoffs 101 beispielhaft näher für den Fall beschrieben werden, dass es sich bei dem zu analysierenden Stoff 101 um menschliches oder tierisches Gewebe handelt und im Rahmen der Analyse des Stoffs eine Glukose- bzw. Blutzuckerspiegelangabe BZA ermittelt werden soll.

Mit der Einrichtung 105 wird ein elektromagnetischer Messstrahl 112, bei dem es sich vorzugsweise um einen Lichtstrahl im sichtbaren Wellenlängenbereich oder um einen Infrarotlichtstrahl handelt, in das optische Medium 108 eingestrahlt; dieser Messstrahl 112 trifft unterhalb des ersten Bereichs 102 der Oberfläche des Gewebes auf die Grenzfläche GF. An der Grenzfläche GF wird der Messstrahl 112 reflektiert und gelangt zur Detektionseinrichtung 106, die den reflektierten Messstrahl 112 misst.

Gleichzeitig werden mit der Anregungs-Lichtquelle 100 eine oder mehrere Anregungsstrahlen SA, bei denen es sich vorzugsweise um Infrarotstrahlen handelt, erzeugt. Die Wellenlänge des oder der Infrarotstrahlen liegt vorzugsweise in einem Bereich zwischen 3 µm und 20 µm, besonders bevorzugt in einem Bereich zwischen 8 µm und 11 µm.

Die Anregungsstrahlen SA werden mit der Einrichtung 104 zur Intensitätsmodulierung intensitäts- bzw. amplitudenmoduliert. in einer Ausführungsform werden mit der Einrichtung 104 zur Intensitätsmodulierung kurze Lichtpulse erzeugt, vorzugsweise mit einer Pulsfrequenz zwischen 1 kHz und 1 MHz oder Pulspakete (Doppel- oder Mehrfachmodulation), vorzugsweise mit einer Frequenz der Einhüllenden von 1-10 kHz.

Die modulierten Anregungsstrahlen SA werden in das optische Medium 108 eingekoppelt und gelangen nach Passieren der Grenzfläche GF in das Volumen 103 innerhalb des Gewebes.

Die Wellenlänge der Anregungsstrahlen SA ist - mit Blick auf die hier beispielhaft erläuterte Blutzuckermessung - vorzugsweise derart gewählt, dass die Anregungsstrahlen SA von Glukose bzw. Blutzucker signifikant absorbiert werden. Zur Messung von Glukose bzw. Blutzucker sind folgende Infrarotwellenlängen besonders gut geeignet (Vakuumwellenlängen): 8,1 µm, 8,3 µm, 8,5 µm, 8,8 µm, 9,2 µm, 9,4 µm und 9,7 µm. Zudem können glucosetolerante Wellenlängen verwendet werden, die von Glucose nicht absorbiert werden, um andere vorhanden Stoffe zu ermitteln und deren Einfluss auf die Messung auszuschließen.

Durch die Absorption der Anregungsstrahlen SA im Gewebe wird im Bereich des Volumens 103 eine lokale Temperaturerhöhung hervorgerufen, die einen Wärmetransport und damit einhergehend Druckwellen in Richtung auf die Grenzfläche GF auslöst; durch die dadurch an der Grenzfläche GF auftretenden Temperatur- und Druckschwankungen werden die Brechzahl bzw. die Deformation, Mikrostruktur und das Reflexionsverhalten im Bereich 102 bzw. im Reflexionsbereich der Grenzfläche GF moduliert und der Strahlengang der Messstrahlen 112 beeinflusst.

Geht man beispielsweise davon aus, dass ohne Anregungsstrahlen SA die Ausrichtung zwischen der Einrichtung 105 und der Detektionseinrichtung 106 optimal ist und eine maximale Empfangsleistung von der Detektionseinrichtung 106 detektiert wird, so kann aufgrund der Absorption der Anregungsstrahlen SA im Bereich des Volumens 103 und aufgrund des Wärmetransports und der Druckwellen eine (zumindest temporäre) Veränderung der Amplitude oder , bei einer periodischen Modulation, der Phase des reflektierten Messstrahls 112 hervorgerufen werden bzw. eine Intensitätsmodulation des reflektierten Messstrahls 112 auftreten. Der Umfang der Intensitätsmodulation hängt von der Wellenlänge der Anregungsstrahlen SA (wegen der nötigen Absorption im Gewebe) und von der Pulsfrequenz der Anregungsstrahlen SA (wegen des Temperaturtransports und der Druckwellen vom Gewebeinneren in Richtung Grenzfläche GF) und den thermischen Eigenschaften der Probe und des Mediums ab.

Die Änderung der Reflexion des Messstrahls 112 bzw. die zeitabhängige Änderung des Reaktionssignals SR wird von der Detektionseinrichtung 106 quantitativ erfasst, und das Detektionsergebnis D gelangt zur Einrichtung 107.

Die Einrichtung 107 kann anhand von vorab durchgeführten Kalibrations- bzw. Vergleichsmessungen, die in einer Ausführungsform in Form von Vergleichstabellen oder Vergleichskurven in einem Speicher 107a der Einrichtung 107 abgespeichert sind, auf die jeweils aktuelle Konzentration von Glukose bzw. Blutzucker innerhalb des Gewebes bzw. innerhalb des Volumens 103 schließen und eine entsprechende Glukose- bzw. Blutzuckerspiegelangabe BZA erzeugen. Die Vergleichstabellen oder Vergleichskurven können beispielsweise auf der Basis von Glukose- oder Blutzuckerwerten erstellt worden sein, die anhand von Blutproben ermittelt worden sind.

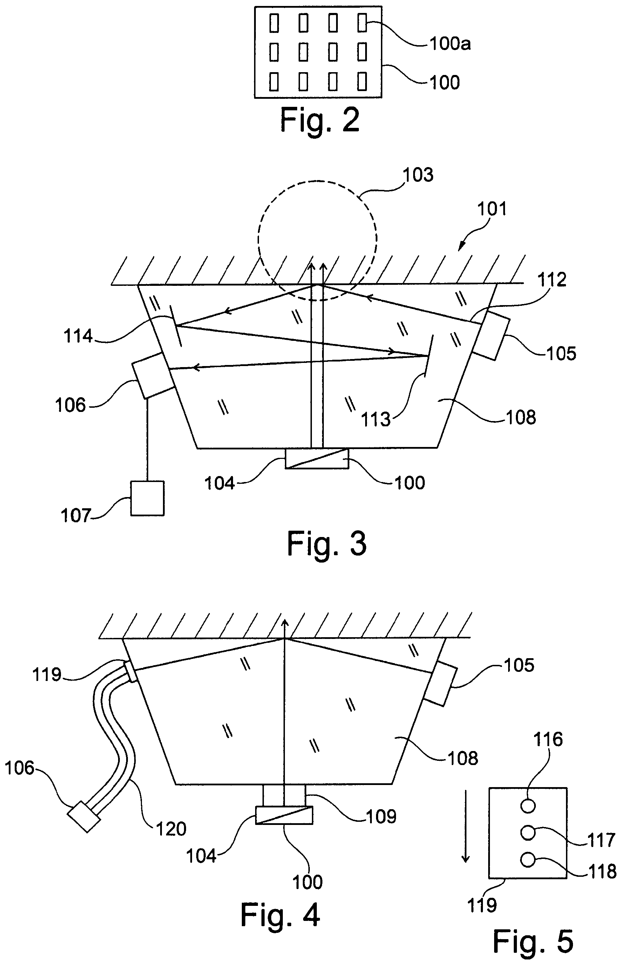

Besonders bevorzugte Ausgestaltungen und Varianten von Vorrichtungen 10 zum Analysieren eines Stoffes 101 werden nachfolgend unter Bezugnahme auf die Figuren 2 bis 10 erläutert.

Die Anregungssendeeinrichtung 100 zum Aussenden des oder der Anregungs-Lichtstrahlen kann als Array ausgebildet sein, wie in der Figur 2 dargestellt ist. Das Array weist wenigstens 5, vorteilhaft wenigstens 10, weiter vorteilhaft wenigstens 15 oder wenigstens 50 oder 100 einzeln ansteuerbare Emitter 100a für jeweils monochromatisches Licht im Absorptionsspektrum eines zu analysierenden Stoffes auf.

Das Array erzeugt vorzugsweise Strahlen mit monochromatischem Licht mit einer oder mehreren, besonders bevorzugt allen der folgenden Wellenlängen (Vakuumwellenlängen): 8,1 µm, 8,3 µm, 8,5 µm, 8,8 µm, 9,2 µm, 9,4 µm und 9,7 µm sowie, falls gewünscht zusätzlich glucosetolerante Wellenlängen.

Die Einrichtung 105 zum Aussenden des Mess-Lichtstrahls 112 und die Detektionseinrichtung 106 können von dem optischen Medium 108 separat angeordnet sein, wie in Figur 1 gezeigt. Mit Blick auf einen minimalen Platzbedarf und minimalen Montageaufwand wird es als vorteilhaft angesehen, wenn die Einrichtung 105 zum Aussenden des Mess-Lichtstrahls 112 und die Detektionseinrichtung 106 unmittelbar an dem optischen Medium 108 angebracht sind, vorzugsweise an gegenüberliegenden Flächenabschnitten 108a und 108b des optischen Mediums 108, wie dies die Figur 3 zeigt.

Es kann vorgesehen sein, dass die Anregungssendeeinrichtung/Anregungs-Lichtquelle 100 unmittelbar oder mittels einer Justagevorrichtung 109 mit dem optischen Medium 108 mechanisch fest verbunden ist. Die Justagevorrichtung 109 ermöglicht vorzugsweise eine Justage des Abstands der Anregungs-Lichtquelle 100 von dem optischen Medium 108 bzw. eine Justage in Strahllängsrichtung und/oder eine Justage in der Ebene senkrecht dazu (vgl. Figur 4).

Wie in den Figuren 3, 4, 6, 7 und 8 gezeigt, kann die Einrichtung 105 zum Aussenden des Mess-Lichtstrahls 112 in den Bereich des optischen Mediums 108 vorgesehen sein, der mit dem ersten Bereich 102 der Stoffoberfläche in Kontakt steht. Eine solche Anordnung ermöglicht das Einstrahlen des Mess-Lichtstrahls 112 in einem flachen Winkel und das Hervorrufen einer Totalreflexion an der Grenzfläche des optischen Mediums 108 zum Stoff 101.

Durch die Einstrahlung in einem flachen (kleinen) Winkel (zur Probeoberfläche) kann die Mirage Deflektion analog zu der bekannten photothermischen ,Bouncing Method' effektiver werden und gleichzeitig die deformationsbedingte Ablenkung des Messstrahls reduziert werden. Der Winkel zwischen der Probeoberfläche und dem Messstrahl kann in einer Ausführungsform kleiner als 20 Grad, kleiner als 10 Grad, insbesondere kleiner als 5 Grad, weiter insbesondere kleiner als 2 Grad oder 1 Grad gewählt werden, um diesen Effekt auszunutzen.

Umgekehrt kann durch die Einstrahlung in steileren (größeren) Winkeln (zur Stoffoberfläche) die Deflektion in Analogie zur der bekannten photothermischen ,Bouncing Method' effektiver werden und gleichzeitig die Mirage-Effekt bedingte Ablenkung des Messstrahls reduziert werden. Der Winkel zwischen der Stoffoberfläche und dem Messstrahl kann in einer Ausführungsform größer als 20 Grad, größer als 30 Grad, insbesondere größer als 45 Grad, weiter insbesondere größer als 60 Grad oder 70 Grad gewählt werden, um diesen Effekt auszunutzen.

Siehe Literatur hierzu:
- M. Bertolotti, G.L. Liakhou, R. Li Voti, S. Paolino, and C. Sibilia. Analysis of the photothermal deflection technique win the surface refection theme: Theory and Experiment. Journal of Applied Physics 83, 966 (1998)

Die Einrichtung 105 zum Aussenden des Mess-Lichtstrahls 112 und/oder die Detektionseinrichtung 106 zur Detektion des Mess-Lichtstrahls 112 bzw. des Reaktionssignals SR können tragend mit dem optischen Medium 108 mechanisch unmittelbar oder mittels einer Justageeinrichtung fest verbunden sein und/oder mittels eines oder mehrerer Lichtwellenleiter 120 an dieses angekoppelt sein.

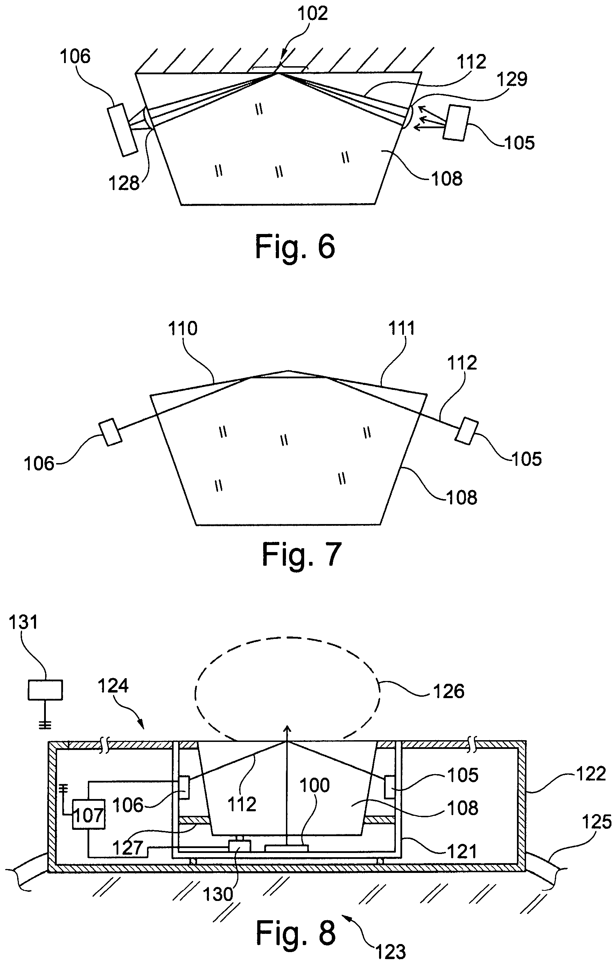

Es kann auch, wie in Figur 6 gezeigt, vorgesehen sein, dass das optische Medium 108 unmittelbar eine Abbildungsoptik 128 und/oder eine Abbildungsoptik 129 (jeweils) in Form einer Linse oder eines anderen Reflektions- oder Beugungsmittels trägt und/oder dass in das optische Medium 108 eine Abbildungs-Optik integriert ist. Die Abbildungsoptik kann jedoch auch in Form einer Linse oder eines anderen Reflexions- oder Beugungselementes in die Anregungssendeeinrichtung oder die Einrichtung zur Erzeugung des Messstrahls integriert sein, beispielsweise, wenn diese als integrierte Bauelemente und/oder als Halbleiterbauelemente ausgebildet sind. Die Abbildungsoptik kann in einer Ausführungsform aus demselben Halbleiterelement durch Ätzen herausgearbeitet sein wie der jeweilige integrierte Schaltkreis, der eine Strahlungsquelle für den Anregungs- oder Messstrahl aufweist.

Es kann auch, wie in Figur 7 gezeigt, vorgesehen sein, dass die Oberfläche des optischen Mediums 108 mehrere gegeneinander geneigte Teilflächen 110, 111 aufweist, an denen der Mess-Lichtstrahl 112 mehrfach reflektiert oder gebrochen wird.

Es kann zudem, wie in Figur 3 dargestellt, vorgesehen sein, dass in oder an dem optischen Medium 108 ein oder mehrere Spiegelflächen 113, 114 zur Reflektion des Mess-Lichtstrahls 112 (und damit des Reaktionssignals SR) vorgesehen sind. Diese Spiegelflächen können durch Inhomogenitäten innerhalb des optischen Mediums 108 oder durch dessen Außenflächen oder durch integrierte/eingepasste/eingegossene oder an dem optischen Medium befestigte, beispielsweise metallische oder metallische beschichtete, Spiegelelemente gebildet sein. Hierdurch wird der optische Weg des Mess-Lichtstrahls 112 in dem optischen Medium 108 bis zum Eintritt in die Detektionseinrichtung 106 verlängert, so dass eine reaktionssignalabhängige Ablenkung des Mess-Lichtstrahls 112 bei der Reflexion an dem Bereich der Oberfläche des Mediums 108, der mit dem ersten Bereich 102 der Stoffoberfläche im Kontakt steht, innerhalb des optischen Mediums 108 vergrößert wird. Die Ablenkung ist dann in der Detektionseinrichtung 106 als absolute Ablenkung nachweisbar.

Die Detektionseinrichtung 106 kann mehrere optisch sensitive Flächen, wie optisch sensitive Halbleiterdioden aufweisen oder auch in einem Anschlusskörper 119 mehrere versetzte Öffnungen 116, 117, 118 (Figur 5), an denen einzelne Lichtwellenleiter 120 (Figur 4) enden, in die das Licht des Messlichtstrahls 112 in Abhängigkeit von seiner Ablenkung eingekoppelt wird. Die Lichtwellenleiter 120 sind dann an einem Anschlusskörper 119, der an dem optischen Medium 108 befestigt sein kann, angeschlossen und leiten das Licht zu dem am Ende der Lichtwellenleiter 120 angeordneten Teil der Detektionseinrichtung 106 (Figur 4). Der Anschlusskörper 119 ist dann, ebenso wie die Lichtwellenleiter 120, auch ein Teil der Detektionseinrichtung 106 zur Detektion des Mess-Lichtstrahls.

Der Vollständigkeit halber soll festgestellt werden, dass auch die Anregungssendeeinrichtung die Anregung ganz oder abschnittsweise mittels eines oder mehrerer Lichtwellenleiter zu der Stoffoberfläche senden kann. in einer Ausführungsform kann die Anregungssendeeinrichtung unmittelbar mit einem oder mehreren Lichtwellenleitern gekoppelt sein die an das optische Medium angekoppelt sind.

Es kann auch, wie in Figur 8 dargestellt, vorgesehen sein, dass die Anregungssendeeinrichtung 100, die Einrichtung 105 zum Aussenden des Mess-Lichtstrahls 112 und die Detektionseinrichtung 106 unmittelbar aneinander oder an einem gemeinsamen Träger 121 befestigt sind. Der Träger kann durch ein Kunststoffteil, eine Leiterplatte oder ein Metallblech gebildet sein, das in einem Gehäuse 122 montiert ist. Der Träger, der in der Figur 8 im Querschnitt U-förmig ausgebildet ist, kann dann das optische Medium 108 in einer Ausführungsform wenigstens teilweise umgeben. Auch das optische Medium kann an dem Träger befestigt sein und gegenüber diesem justiert werden.

Der Träger kann auch durch das Gehäuse 122 selbst oder ein Gehäuseteil gebildet sein.

Es kann auch vorgesehen sein, dass die Vorrichtung mit dem Gehäuse 122 am Körper 123 einer Person befestigbar ist, wobei die Anregungssendeeinrichtung 100 zum Aussenden eines oder mehrerer Anregungs-Lichtstrahlen SA, die Einrichtung 105 zum Aussenden des Mess-Lichtstrahls 112 und die Detektionseinrichtung 106 zur Detektion des zeitabhängigen Reaktionssignals SR derart angeordnet und eingerichtet sind, dass die zur Messung geeignete Seite (mit einem für die Anregungsstrahlung transparenten Messfenster) der Vorrichtung auf der dem Körper abgewandten Seite der Vorrichtung liegt, so dass der zu analysierende Stoff auf der dem Körper 123 abgewandten Seite 124 des Gehäuses 122 vermessbar ist. Hierzu ist in der Figur 8 dargestellt, dass das Gehäuse 122 mittels eines zum Gehäuse gehörenden Riemens 125 am Körper 123 einer Person, in einer Ausführungsform in Form eines Armbandes an einem Handgelenk befestigt ist. Auf der dem Handgelenk abgewandten Seite 124 weist dann das Gehäuse ein für den Anregungs-Lichtstrahl SA durchlässiges Fenster auf, oder das optische Medium 108 ist unmittelbar in die nach außen weisende Seite 124 des Gehäuses eingepasst und bildet selbst abschnittsweise die Oberfläche des Gehäuses.

Wie in Figur 8 dargestellt, kann dann eine gestrichelt schematisiert dargestellte Fingerbeere 126 auf das optische Medium 108 aufgelegt und vermessen werden.

Das optische Medium 108 kann innerhalb des Gehäuses 122, ebenso wie der Träger 121, oder unmittelbar an dem Gehäuse 122 befestigt sein. Das optische Medium 108 kann auch unmittelbar mit dem Träger 121 verbunden sein, wobei eine Justageeinrichtung 127 zur relativen Positionierung des Trägers 121 zum optischen Medium 108 vorgesehen sein sollte.

Es ist auch denkbar, die Anregungs-Lichtquelle 100, die Einrichtung 105 und die Detektionseinrichtung 106 oder auch nur eines oder zwei der genannten Elemente unmittelbar an dem optischen Medium 108 und das oder die jeweils anderen Elemente an dem Träger 121 zu befestigen.

Durch das optische Fenster in dem Gehäuse 122 und/oder durch das optische Medium 108 hindurch können auch andere Parameter der Stoffoberfläche bzw. der aufgelegten Fingerbeere 126 vermessbar sein, wie in einer Ausführungsform ein Fingerabdruck. Hierzu kann in dem Gehäuse ein optischer Detektor 130 in Form einer Kamera beispielsweise auch an dem Träger 121 befestigt sein, die durch das optische Medium 108 hindurch ein Bild der Stoffoberfläche digital aufnimmt. Dieses Bild wird innerhalb einer Verarbeitungseinrichtung 107, die unmittelbar mit der Detektionseinrichtung und auch mit der Anregungssendeeinrichtung verbunden sein kann ebenso verarbeitet wie die Messinformationen von der Detektionseinrichtung 106. Die Verarbeitungseinrichtung kann auch Steuerungsaufgaben der Messung übernehmen. Sie kann auch wenigstens teilweise von den übrigen Teilen der Vorrichtung getrennt und entfernt sein und mittels einer Funkverbindung mit diesen kommunizieren.

Die Bilddaten von der Kamera 130 können somit innerhalb des Gehäuses oder über eine Funkverbindung auch außerhalb des Gehäuses weiterverarbeitet und mit einer Personenidentitäts-Datenbank verglichen werden, um Kalibrationsdaten der identifizierten Person abzurufen.

Derartige Kalibrationsdaten können auch entfernt (remote) in einer Datenbank, in einer Ausführungsform einer Cloud, abrufbar gespeichert sein. Auch die Messdaten der Detektionseinrichtung 106 können sowohl innerhalb als auch außerhalb des Gehäuses weiterverarbeitet werden.

Werden Daten außerhalb des Gehäuses verarbeitet, so sollten die Ergebnisdaten vorzugsweise zu der Vorrichtung innerhalb des Gehäuses zurückgefunkt werden, um dort angezeigt werden zu können.

In jedem Fall kann eine Anzeige an dem Gehäuse 122 vorgesehen sein, die vorteilhaft durch das optische Fenster, in einer Ausführungsform auch teilweise durch das optische Medium ablesbar ist. Die Anzeige kann auch durch das optische Fenster eine Leuchtanzeige auf eine Anzeigefläche projizieren und zu diesem Zweck eine Projektionsvorrichtung aufweisen. Durch die Anzeige kann in einer Ausführungsform ein Mess- oder Analyseergebnis, insbesondere eine Glucosekonzentration angezeigt werden. Die Ausgabe kann in einer Ausführungsform über einen Zeichen- oder Farbcode erfolgen. Es kann durch die Anzeige oder eine zu dieser parallele Signaleinrichtung in einer Ausführungsform ein Vorschlag für eine Insulindosis abhängig von weiteren Patientenparametern (z.B. Insulinkorrekturfaktor) oder eine automatische Signalübertragung an eine Dosiereinrichtung in Form einer Insulinpumpe erfolgen.

Die Verbindung der Vorrichtung von und zu einer externen Datenverarbeitungseinrichtung 131 kann durch alle gängigen Standards, wie Lichtleiter, Kabel, Funk (z. B. Bluetooth, WiFi), oder auch Ultraschall oder Infrarotsignale realisiert sein.

Die Figur 9 zeigt eine Modulationseinrichtung mit einer die Anregungssendeeinrichtung 100 moduliert ansteuernden Steuerung 132. Sowohl die Steuerung 132 als auch die Detektionseinrichtung 106 für den Mess-Lichtstrahl sind mit der Auswertungseinrichtung 107 verbunden.

Figur 10 zeigt eine Anregungs-Lichtquelle 100, vor der eine durch ein MEMS (Mikro-elektromechanisches System) 135 angetriebene Spiegeleinrichtung mit einem oder mehreren Mikrospiegeln 133, 134, wie sie z. B. aus der optischen Bild-Projektortechnik bekannt sind, zur zeitweiligen Umlenkung des Anregungslichtstrahls in eine Umlenkrichtung 136 angeordnet ist.

Die Figur 11 zeigt eine Anregungs-Lichtquelle 100, vor der eine optische, bezüglich der Transmission mittels einer Steuereinrichtung 137 steuerbare Schicht 138 im Anregungslichtstrahl, in einer Ausführungsform mit LCD-Zellen angeordnet ist.

Die vorliegende Schutzrechtsanmeldung bezieht sich (wie einleitend bereits erwähnt) neben den oben beschriebenen Anspruchsgegenständen und Ausführungsbeispielen auch auf die folgenden Aspekte. Diese Aspekte können einzeln oder in Gruppen jeweils mit Merkmalen der Ansprüche kombiniert werden. Die Aspekte stellen darüber hinaus auch jeweils für sich genommen oder miteinander oder mit Anspruchsgegenständen kombiniert eigenständige Erfindungen dar. Die Anmelderin behält sich vor, diese Erfindungen zu einem späteren Zeitpunkt zum Gegenstand von Ansprüchen zu machen. Dies kann im Rahmen dieser Anmeldung oder auch im Rahmen von späteren Teilanmeldungen oder Nachanmeldungen unter Inanspruchnahme der Priorität dieser Anmeldung geschehen:
1) Verfahren zum Analysieren eine Stoffes in einem Körper, umfassend:
   - Aussenden eines Anregungs-Lichtstrahls mit einer oder mehreren spezifischen Anregungswellenlängen durch einen ersten Bereich der Oberfläche des Körpers,
   - Intensitätsmodulation des Anregungs-Lichtstrahls mit einer oder mehreren Frequenzen, insbesondere nacheinander, durch ein von einem mechanischen Chopper verschiedenes Bauteil, insbesondere durch eine elektronische Ansteuerung der Anregungs-lichtquelle, eine Verstelleinrichtung für einen Resonator eines als Anregungslichtquelle dienenden Anregungslasers oder eine bewegliche Spiegeleinrichtung, eine steuerbare Beugungseinrichtung, eine Verschluss- oder Spiegeleinrichtung, die an einem Motor, wie einem Steppermotor, oder mit einem MEMS gekoppelt ist oder eine bezüglich der Transmission steuerbare Schicht im Strahlengang,
   - mittels eines außerhalb des Körpers angeordneten Detektors die zeitaufgelöste Detektion eines Reaktionssignals, das auf die Wirkung der wellenlängenabhängigen Absorption des Anregungs-Lichtstrahls im Körper zurückgeht.

Die Modulation kann in einer Ausführungsform durch Interferenz oder Beeinflussung der Phase oder Polarisation der Strahlung der Anregungssendeeinrichtung erfolgen, insbesondere, wenn diese eine Laserlichteinrichtung umfasst.
2) Verfahren nach Aspekt 1, dadurch gekennzeichnet, dass der Anregungslichtstrahl durch mehrere Emitter oder Multiemitter, insbesondere in Form eines Laser-Arrays, erzeugt wird, die Licht mit unterschiedlichen Wellenlängen gleichzeitig oder nacheinander oder in beliebigen Pulsmustern aussenden.
3) Verfahren nach Aspekt 1 oder 2, dadurch gekennzeichnet, dass an dem ersten Bereich der Oberfläche des Körpers ein akustisches Reaktionssignal durch einen akustischen Sensor erfasst wird.
4) Verfahren nach einem der Aspekte 1 bis 3, dadurch gekennzeichnet, dass an dem ersten Bereich der Oberfläche des Körpers ein Reaktionssignal durch einen Infrarotstrahlungssensor, insbesondere ein Thermoelement, ein Bolometer oder ein Halbleiterdetektor, zum Beispiel ein Quantum-Cascade-Detektor, erfasst wird.
5) Verfahren nach einem der Aspekte 1 bis 4, umfassend die Schritte:
   - Herstellen des Kontakts eines optischen Mediums mit einer Stoffoberfläche, so dass zumindest ein Bereich der Oberfläche des optischen Mediums in Kontakt mit dem ersten Bereich der Oberfläche des Körpers steht;
   - Aussenden eines Anregungs-Lichtstrahls mit einer Anregungswellenlänge in ein in dem Stoff unterhalb des ersten Bereiches der Oberfläche liegendes Volumen insbesondere durch den Bereich der Oberfläche des optischen Mediums hindurch, der in Kontakt mit dem ersten Bereich der Stoffoberfläche steht,
   - Messen der Temperatur in dem ersten Bereich der Oberfläche des optischen Mediums durch ein optisches pyrometrisches Verfahren,
   - Analysieren des Stoffes anhand der detektierten Temperaturerhöhung in Abhängigkeit von der Wellenlänge des Anregungs-Lichtstrahls.
6) Verfahren nach Aspekt 5, gekennzeichnet durch
   das Aussenden eines Mess-Lichtstrahls durch das optische Medium auf den Bereich der Oberfläche, des optischen Mediums, der in direktem Kontakt mit der Stoffoberfläche steht, derart, dass der Mess-Lichtstrahl und der Anregungslichtstrahl auf der Grenzfläche des optischen Mediums und der Stoffoberfläche, an der der Mess-Lichtstrahl reflektiert wird, einander unmittelbar benachbart sind oder überlappen;
   Direktes oder indirektes Detektieren einer Ablenkung des reflektierten Mess-Lichtstrahls in Abhängigkeit von der Wellenlänge des Anregungs-Lichtstrahls und
   Analysieren des Stoffes anhand der detektierten Ablenkung des Mess-Lichtstrahls in Abhängigkeit von der Wellenlänge des Anregungs-Lichtstrahls.
7) Verfahren nach einem der Aspekte 5 oder 6, dadurch gekennzeichnet, dass der Mess-Lichtstrahl durch dieselbe Lichtquelle erzeugt wird, die den Anregungs-Lichtstrahl erzeugt.
8) Verfahren nach einem der Aspekte 5, 6 oder 7, dadurch gekennzeichnet, dass der Mess-Lichtstrahl nach der Ablenkung und vor der Detektion innerhalb des optischen Mediums, außerhalb des optischen Mediums oder teilweise innerhalb und teilweise außerhalb des optischen Mediums ein- oder mehrmals reflektiert wird.
9) Verfahren nach Aspekt 1 oder einem der anderen vorangehenden bzw. folgenden, dadurch gekennzeichnet, dass der Mess-Lichtstrahl ein intensitätsmodulierter, insbesondere gepulster Anregungs-Lichtstrahl insbesondere im Infrarot-Spektralbereich ist, wobei insbesondere die Modulationsrate zwischen 1 Hz und 10 kHz, vorzugsweise zwischen 10Hz und 3000Hz liegt.
10) Verfahren nach Aspekt 1 oder einem der anderen vorangehenden bzw. folgenden, dadurch gekennzeichnet, dass das Licht des/der Anregungs- Lichtstrahlen durch eine integrierte Anordnung mit mehreren Einzellasern, insbesondere einem Laserarray, gleichzeitig oder nacheinander oder teilweise gleichzeitig und teilweise nacheinander erzeugt wird.
11) Verfahren nach Aspekt 1 oder einem der anderen vorangehenden bzw. folgenden, dadurch gekennzeichnet, dass aus den bei verschiedenen Modulationsfrequenzen des Anregungs-Lichtstrahls gewonnenen Reaktionssignalen eine Intensitätsverteilung der Reaktionssignale in Abhängigkeit von der Tiefe unter der Oberfläche ermittelt wird, in der die Reaktionssignale entstehen.
12) Verfahren nach Aspekt 1 oder einem der anderen vorangehenden bzw. folgenden, dadurch gekennzeichnet, dass aus der Phasenlage der Reaktionssignale im Verhältnis zu einem modulierten Anregungs- Lichtstrahl bei einer oder verschiedenen Modulationsfrequenzen des Anregungs-Lichtstrahls eine Intensitätsverteilung der Reaktionssignale in Abhängigkeit von der Tiefe unter der Oberfläche ermittelt wird, in der die Reaktionssignale entstehen.
13) Verfahren nach Aspekt 11 oder 12, dadurch gekennzeichnet, dass zur Ermittlung der Intensitätsverteilung der Reaktionssignale in Abhängigkeit von der Tiefe unter der Oberfläche die Messergebnisse zu verschiedenen Modulationsfrequenzen gewichtet und miteinander verknüpft werden.
14) Verfahren nach Aspekt 11, 12 oder 13, dadurch gekennzeichnet, dass aus der gewonnenen Intensitätsverteilung über die Tiefe unter der Oberfläche des Körpers eine Stoffdichte eines den Anregungs- Lichtstrahl in spezifischen Wellenlängenbereichen absorbierenden Stoffes in einer bestimmten Tiefe oder einem Tiefenbereich ermittelt wird.
15) Verfahren nach Aspekt 1 oder einem der anderen vorangehenden bzw. folgenden, dadurch gekennzeichnet, dass im unmittelbar vor oder nach oder während der Detektion des Reaktionssignals/der Reaktionssignale wenigstens eine biometrische Messung an dem Körper in dem ersten Bereich der Oberfläche oder unmittelbar diesem benachbart, insbesondere eine Vermessung eines Fingerabdrucks durchgeführt und der Körper, insbesondere eine Person identifiziert wird und dass insbesondere der Detektion der Reaktionssignale Referenzwerte (Kalibrierungswerte) zugeordnet werden.
16) Vorrichtung zum Analysieren eines Stoffes,
   mit einer Einrichtung zum Aussenden eines oder mehrerer Anregungs-Lichtstrahlen mit jeweils einer Anregungswellenlänge in ein in dem Stoff unterhalb eines ersten Bereiches seiner Oberfläche liegendes Volumen, mit einer Einrichtung zum Modulieren eines Anregungslichtstrahls, die durch eine Modulationseinrichtung der Strahlungsquelle, insbesondere ihrer Ansteuerung, eine Interferenzeinrichtung, eine Phasen- oder Polarisationsmoduliereinrichtung und/oder wenigstens einen, im Strahlengang angeordneten gesteuerten Spiegel, und/oder eine bzgl. ihrer Transparenz steuerbare, im Strahlengang angeordnete Schicht gebildet ist, sowie mit einer Detektionseinrichtung zur Detektion eines zeitabhängigen Reaktionssignals in Abhängigkeit von der Wellenlänge des Anregungslichts und der Intensitäts- Modulation des Anregungslichts und mit einer Einrichtung zum Analysieren des Stoffes anhand der detektierten Reaktionssignale.
17) Vorrichtung nach Aspekt 16 mit einer Einrichtung zur Ermittlung von Reaktionssignalen gesondert nach verschiedenen Intensitäts-Modulationsfrequenzen und/oder mit einer Einrichtung zur Ermittlung von Reaktionssignalen in Abhängigkeit von der Phasenlage des jeweiligen Reaktionssignals relativ zur Phase der Modulation des Anregungs- Lichtstrahls, insbesondere in Abhängigkeit von der Modulationsfrequenz des Anregungs-Lichtstrahls.
18) Vorrichtung zum Analysieren eine Stoffes nach Aspekt 16 oder 17, mit einem optischen Medium zum Herstellen des Kontakts der Oberfläche des optischen Mediums mit einem ersten Bereich der Stoffoberfläche, sowie mit
   einer Einrichtung zum Aussenden eines Anregungs-Lichtstrahls mit einer oder mehreren Anregungswellenlänge in ein in dem Stoff unterhalb der des ersten Bereiches der Oberfläche liegendes Volumen insbesondere durch den Bereich der Oberfläche des optischen Mediums hindurch, der in Kontakt mit der Stoffoberfläche steht, sowie mit einer Einrichtung zum
   Messen der Temperatur in dem Bereich der Oberfläche des optischen Mediums, der mit dem ersten Bereich der Stoffoberfläche im Kontakt steht durch ein optisches Verfahren, und mit einer Einrichtung zum Analysieren des Stoffes anhand der detektierten Temperaturänderungen in Abhängigkeit von der Wellenlänge des Anregungs-Lichtstrahls und der Intensitätsmodulation des Anregungs- Lichtstrahls.
19) Vorrichtung nach Aspekt 18, dadurch gekennzeichnet, dass die Anregungs-Lichtquelle unmittelbar mit dem optischen Medium mechanisch fest verbunden ist.
20) Vorrichtung nach Aspekt 18, dadurch gekennzeichnet, dass eine Einrichtung zum Aussenden eines Mess-Lichtstrahls in den Bereich des optischen Mediums vorgesehen ist, der mit dem ersten Bereich der Stoffoberfläche im Kontakt steht und dass diese Einrichtung/oder die Detektionseinrichtung zur Detektion des Mess- Lichtstrahls unmittelbar mit dem optischen Medium mechanisch fest verbunden und/oder mittels eines Lichtwellenleiters an dieses angekoppelt ist.
21) Vorrichtung nach Aspekt 18, 19 oder 20, dadurch gekennzeichnet, dass das optische Medium unmittelbar eine Abbildungsoptik trägt und/oder dass in das optische Medium eine Abbildungs-Optik integriert ist.
22) Vorrichtung nach Aspekt 18 oder einem der anderen vorangehenden bzw. folgenden, dadurch gekennzeichnet, dass die Oberfläche des optischen Mediums mehrere gegeneinander geneigte Teilflächen aufweist, an denen der Mess-Lichtstrahl mehrfach reflektiert wird.
23) Verfahren nach Aspekt 18 oder einem der anderen vorangehenden bzw. folgenden, dadurch gekennzeichnet, dass in oder an dem optischen Medium ein oder mehrere Spiegelflächen zur Reflektion des Mess- Lichtstrahls vorgesehen sind.
24) Vorrichtung nach Aspekt 16 oder 17, dadurch gekennzeichnet, dass die Detektionseinrichtung zur Detektion eines zeitabhängigen Reaktionssignals einen akustischen Detektor zur Erfassung akustischer Wellen an der Stoffoberfläche, insbesondere mit einem Resonator, weiter insbesondere mit einem Helmholtzresonator. Als Detektor der akustischen Quelle dient eine Quartzgabel mit vorzugsweise der gleichen Resonanzfrequenz wie der Resonator. Der Resonator kann offen oder geschlossen sein. Die Quartzgabel ist vorzugsweise im oder am Hals des Resonantors (off-beam) oder inner-/außerhalb des Resonators (in-beam) angeordnet.
25) Vorrichtung nach Aspekt 16, 17 oder 18, dadurch gekennzeichnet, dass die Detektionseinrichtung zur Detektion eines zeitabhängigen Reaktionssignals einen Wärmestrahlungs- Detektor zur Erfassung der Wärmestrahlung an der Stoffoberfläche, insbesondere einen Infrarotdetektor, weiter insbesondere ein Thermoelement, ein Bolometer, oder einen Halbleiterdetektor aufweist.
26) Vorrichtung nach einem der Aspekte 16 bis 25, dadurch gekennzeichnet, dass die Anregungs-Lichtquelle und die Detektionseinrichtung unmittelbar aneinander oder an einem gemeinsamen Träger befestigt sind, der insbesondere durch ein Gehäuse oder Gehäuseteil der Vorrichtung gebildet ist.
27) Vorrichtung nach einem der Aspekte 16 bis 26, dadurch gekennzeichnet, dass die Vorrichtung ein tragbares Gehäuse aufweist, das am Körper einer Person befestigbar ist, wobei die Einrichtung zum Aussenden eines oder mehrerer Anregungs-Lichtstrahlen und die Detektionseinrichtung zur Detektion eines zeitabhängigen Reaktionssignals derart angeordnet und eingerichtet ist, dass der zu analysierende Stoff auf der dem Körper abgewandten Seite des Gehäuses vermessen wird.
28) Vorrichtung nach einem der Aspekte 16 bis 26, dadurch gekennzeichnet, dass die Vorrichtung ein tragbares Gehäuse aufweist, das am Körper einer Person befestigbar ist und dass das Gehäuse der Vorrichtung ein für den Anregungs- Lichtstrahl durchlässiges Fenster auf seiner bei der vorgesehenen Trageposition vom Körper abgewandten Seite aufweist.
29. Vorrichtung zum Analysieren eines Stoffes mit einer Anregungssendeeinrichtung zum Erzeugen mindestens eines elektromagnetischen Anregungsstrahls, insbesondere Anregungs-Lichtstrahls, mit zumindest einer Anregungswellenlänge, einer Detektionseinrichtung zur Detektion eines Reaktionssignals und einer Einrichtung zum Analysieren des Stoffes anhand des detektierten Reaktionssignals.
30. Vorrichtung nach einem der vorhergehenden Aspekte 16 bis 29, dadurch gekennzeichnet, dass die Detektionseinrichtung zur Messung der Deformation eines Kristalls eingerichtet ist. Die Deformation kann analog zur photothermischen ,Bouncing Method' durch die Wahl von steileren (größeren) Einfallswinkeln des Messstrahls zur Probeoberfläche effektiver gemessen werden und der Einfluss der Mirage-Effekt bedingten Ablenkung des Messstrahls minimiert werden.

### Literatur:

M. Bertolotti, G.L. Liakhou, R. Li Voti, S. Paolino, and C. Sibilia. Analysis of the photothermal deflection technique win the surface refection theme: Theory and Experiment. Journal of Applied Physics 83, 966 (1998)

Der Cantilever kann entweder direkt auf der Probe platziert werden oder auf einem hinreichend dünnen optischen Medium auf welchem die Probe auf der einen Seite und der Cantilever auf der gegenüberliegenden Seite platziert wird. Durch die thermische Expansion der Probe bzw. des optischen Elements wird der Cantilever durch die Absorption des modulierten Pumpstrahls bedingte thermische Expansion in Schwingung versetzt. Der Messstrahl wird auf die an der oberen Seite der Spitze des Cantilevers reflektiert und bedingt durch die Schwingung abhängig von der eingestrahlt Wellenlänge und den thermischen Eigenschaften der Probe, sowie der Modulationsfrequenz abgelenkt. Diese Ablenkung wird detektiert.
31. Vorrichtung nach einem der vorhergehenden Aspekte 16 bis 30, dadurch gekennzeichnet, dass die Anregungssendeeinrichtung einen Abfragelaser oder eine LED, beispielsweise eine NIR(near-infrared)-LED, enthält.
32. Vorrichtung nach einem der vorhergehenden Aspekte 16 bis 31, dadurch gekennzeichnet, dass die Anregungssendeeinrichtung einen Probe-Laser aufweist, der einen kleineren Durchmesser als ein zusätzlicher Pump-Laser hat.
33. Vorrichtung nach einem der vorhergehenden Aspekte 16 bis 32, dadurch gekennzeichnet, dass zur Erzielung eines günstigeren Signal-/Rausch-Verhältnis eine spezielle Beschichtung, insbesondere des Emitters, z. B. IRE, vorgesehen ist, so dass Wärme besser abgeführt wird (z. B. "Wärmeleitpaste").

Das optische Element kann an der Kontaktfläche derartig beschichtet sein, dass eine verbesserte Ableitung des thermischen Signals in das optische Medium erfolgen kann. Zudem kann die Beschichtung des weiteren auch als Kratzschutz dienen, und auch durch geschickte Materialwahl eine reflektierende Oberfläche für den Messstrahl darstellen. Hierbei muss die Transparenz für das Anregungslicht zwingend gegeben bleiben.
34. Vorrichtung nach einem der vorhergehenden Aspekte 16 bis 33, dadurch gekennzeichnet, dass die Vorrichtung eine Einrichtung für
   i. Pulstrains / Doppelmodulation
   ii. Schwingspiegel
   iii. MEMS Interferometer
   aufweist.
35. Vorrichtung nach einem der vorhergehenden Aspekte 16 bis 34, dadurch gekennzeichnet, dass die Vorrichtung für eine Person am Körper in einer Ausführungsform durch eine mit dem Gehäuse verbundene Halteeinrichtung wie einen Riemen, ein Band oder eine Kette oder eine Spange, dauerhaft tragbar ausgeführt ist und/ oder die Detektionseinrichtung eine Detektionsfläche aufweist, die auch als Anzeigefläche für Informationen wie Messwerte, Uhrzeiten und/ oder Textinformationen nutzbar ist.
36. Vorrichtung nach dem vorhergehenden Aspekt 35, dadurch gekennzeichnet, dass die Vorrichtung eine Abziehfolie im Bereich der Detektionsfläche, vorzugsweise neben der Detektionsfläche, zur Vorbehandlung der Stoffoberfläche und Sicherstellung einer sauberen Oberfläche und/ oder in einer Ausführungsform im Fall der Glucosemessung konkret zur Hautreinigung aufweist.
37. Vorrichtung nach einem der vorhergehenden Aspekte 16 bis 36, dadurch gekennzeichnet, dass die Detektionseinrichtung eingerichtet ist zum Lesen und Erkennen von Fingerabdrücken zum Abruf bestimmter Werte/ Kalibrierungen einer Person und/ oder zur Erkennung der Lage eines Fingers, vorzugsweise zum Erkennen und Bestimmen einer ungewollten Bewegung während der Messung, aufweist.
38. Vorrichtung nach einem der vorhergehenden Aspekte 16 bis 37, dadurch gekennzeichnet, dass die Detektionseinrichtung eine Ergebnisanzeige aufweist, die vorzugsweise mit Farbkodierung, als analoge Anzeige, in einer Ausführungsform inkl. Fehleranzeige (beispielhaft: "100mg/dl plus/minus 5mg/dl"), akustisch und/ oder mit Ergebnisanzeige von Messwerten in größeren Schritten als die Messgenauigkeit der Vorrichtung es erlaubt ausgeführt ist. Hierdurch werden z.B. kleine Schwankungen, die einen Benutzer verunsichern könnten, nicht mitgeteilt.
39. Vorrichtung nach einem der vorhergehenden Aspekte 16 bis 38, dadurch gekennzeichnet, dass die Vorrichtung zum Austausch gemessener Daten sowie zum Abruf von Kalibrier- oder anderen Daten von anderen Geräten oder Cloud-Systemen Datenschnittstellen aufweist,
   wobei die Vorrichtung vorzugsweise so eingerichtet ist, dass die Datenübertragung verschlüsselt, insbesondere durch Fingerabdruck oder andere biometrische Daten des Bedieners verschlüsselt, erfolgen kann.
40. Vorrichtung nach einem der vorhergehenden Aspekte 16 bis 39, dadurch gekennzeichnet, dass die Vorrichtung so eingerichtet ist, dass ein Vorschlag für eine der Person zu gebende Insulindosis durch die Vorrichtung im Zusammenspiel mit anderen Daten ermittelbar ist (z.B. Insulinkorrekturfaktor) und/oder Gewicht, Körperfett mitmessbar und/oder manuell angebbaren oder von anderen Geräten an die Vorrichtung übermittelbar ist.
41. Vorrichtung nach einem der vorhergehenden Aspekte 16 bis 40 dadurch gekennzeichnet, dass die Vorrichtung zur Erhöhung der Messgenauigkeit eingerichtet ist zur Ermittlung weiterer Parameter, in einer Ausführungsform mittels Sensoren zum Ermitteln der Hauttemperatur, Diffusivität, Leitfähigkeit/ Feuchtigkeit der Haut, zur Messung der Polarisation des Lichtes (Ausschluss von Wasser/Schweiß auf der Fingeroberfläche) oder dergleichen.

Wasser und Schweiß auf der Hautoberfläche eines Menschen, die die Glucosemessung beeinflussen können, können durch eine Test- Anregung mit einer Anregungsstrahlung durch die Anregungssendeinrichtung mit den wasserspezifischen Banden bei 1640 cm-1 (6.1 µm) und 690 cm-1 (15 µm) detektiert werden. Sollte die Absorption einen bestimmten Wert überschreiten ist der Messort/die Stoffoberfläche/Hautoberfläche zu nass für eine zuverlässige Messung. Alternativ kann die Leitfähigkeit des Stoffes in der Nähe oder direkt an der Messstelle gemessen werden, um die Feuchtigkeit zu bestimmen. Es kann dann eine Fehlermeldung sowie eine Anweisung zum Trocknen ausgegeben werden.
42. Vorrichtung nach einem der vorhergehenden Aspekte 16 bis 41 dadurch gekennzeichnet, dass die Vorrichtung über eine Abdeckung im Strahlengang des Pump- und/oder Messstrahl-Lasers verfügt. Hierdurch kann für die obligatorische Augensicherheit von Lebewesen gesorgt werden.
43. Vorrichtung nach einem der vorhergehenden Aspekte 16 bis 42 dadurch gekennzeichnet, dass die Vorrichtung über eine wechselbare Detektionsfläche verfügt.
44. Vorrichtung nach einem der vorhergehenden Aspekte 16 bis 43 dadurch gekennzeichnet, dass die Vorrichtung als optisches Medium über einen stellenweise geriffelten oder aufgerauhten Kristall verfügt, der der Probe (z.B. dem Finger) einen bessere Justierung ermöglicht. Die Messstelle, auf die die zu analysierende Stoffoberfläche aufgelegt wird, ist vorzugsweise ohne Riffelung und glatt ausgebildet.
45. Vorrichtung nach einem der vorhergehenden Aspekte 16 bis 44 dadurch gekennzeichnet, dass für den Messstrahl eine zylindrische TEMpl TEM00 Mode oder anstelle der zylindrischen TEMpl TEM00 Mode andere Moden TEM01 (Dougnut) TEM02 oder TEM03 verwendet werden. Besonders die letzteren haben den Vorteil das ihre Intensität auf das Sensitivitäts-Profil der Quadrantendiode abgestimmt werden können, die den Detektor für den abgelenkten Messstrahl darstellt (siehe Figuren). Desweiteren können rechteckige Moden TEMmn benutzt werden wie TEM30 oder TEM03 oder höher. Damit können Abtast/Messstrahlen genutzt werden die in horizontaler oder vertikaler Richtung Störungsunempfindlicher sind.
46. Vorrichtung nach einem der vorhergehenden Aspekte 16 bis 45 dadurch gekennzeichnet, dass die Vorrichtung nicht nur an einem Punkt sondern in einem Raster misst. Dies kann entweder durch eine Verschiebung des Pump- oder des Probe-Lasers oder der Detektionseinheit geschehen. Anstatt einer Verschiebung ist auch ein oder mehrere Arrays von Pump- oder Probe-Lasern denkbar.

Weitere Detektionsmethoden zum Nachweis eines Reaktionssignals nach Aussendung eines Anregungsstrahls können umfassen: - Photoakustische Detektion - photoakustische Detektion mittels einer Stimmgabel oder eines anderen Schwingungselementes oder: eine leicht abgewandelte Form der Photoakustik mit offener QePAS-Zelle (Quartz enhanced PhotoAcustic Spectroskopy)). Durch diese Methoden können Druckschwankungen/schwingungen an der Stoffoberfläche nachgewiesen und ebenso ausgewertet werden, wie oben für die gemessene Strahlablenkung beschrieben.

Grundsätzlich können zur Tiefenprofilierung ermittelte Werte einer Phasenverschiebung des Reaktionssignals gegenüber einer periodischen Modulation des Anregungsstrahls genutzt werden. (Erwärmungs-/Abkühlphasen der Stoffoberfläche sollten dazu genauer bzgl. ihres Verlaufes ausgewertet werden).

Mit der beschriebenen Vorrichtung kann ein Vorrat von Klebestreifen zum Abtragen toter Hautschichten verbunden sein, um eine möglichst störungsfreie Messung an einem menschlichen Körper zu erlauben, sowie Pflaster mit Wärmeleitpaste, die auf das optische Medium regelmäßig aufgebracht werden können. Das optische Medium kann bei geeigneter Befestigung und Justierung der übrigen Teile austauschbar sein.

Die Vorrichtung kann zur Messung nicht nur an einem Finger einer Person, sondern auch an einer Lippe oder einem Ohrläppchen vorgesehen und eingerichtet sein.

Die Messung kann in einigen Ausführungen auch ohne direkte Berührung und Auflage des Fingers oder eines anderen Körperteils (auf Entfernung) funktionieren, wodurch ein kontaktloses Messen ermöglicht wird.

Die Messung kann durch Kombination mehrerer der geschilderten und erläuterten Messsysteme mit ähnlicher Fehleranfälligkeit bezüglich der Genauigkeit und Zuverlässigkeit verbessert werden.

DAQ und LockIn-Verstärker in der Auswertung können in einem Gerät zusammengefasst werden und die Auswertung kann insgesamt digitalisiert werden.

Die Messung kann mit der Vorrichtung auch an einer bewegten Stoffoberfläche durchgeführt werden, so dass im Zuge einer Rastermessung: Anregungslichtquelle und- und/oder Messlichtquelle während der Messung in einem Raster über die Haut fahren, sich ggf. Hautunregelmäßigkeiten kompensieren oder wegmitteln lassen.

Die Sensitivität der Detektionseinrichtung/Deflektionseinheit kann durch Einstellung /Variation der Wellenlänge des Probe-Beams/der Messlichtquelle optimiert werden. Hierzu kann die Messlichtquelle bzgl. der Wellenlänge veränderbar sein oder mehrere Laserlichtquellen verschiedener Wellenlänge zur Auswahl oder Kombination enthalten.

Für die Ablenkung des Pump-/Probe-Lasers kann ein optimaler transversaler Mode (TEM) gewählt werden.

Anregungssendeeinrichtung" Messlichtquelle und Detektor können als ein gemeinsamer Array aufgebaut werden und die strahlen können im optischen Medium geeignet umgelenkt werden, um Aussendung und Empfang aller Strahlen auf eine Stelle zu konzentrieren.

Eine Linse auf oder im Kristall des optischen Mediums kann dazu beitragen, den Messlichtstrahl Reaktionssignalabhängig stärker abzulenken.

Zudem ist die Verwendung einer gapfree Photodiode zur Detektion denkbar, dann könnte eine Linse den Messlichtstrahl nach Austritt bündeln und so eine genauere Messung ermöglichen.

Eine zusätzliche Ausgestaltung der Erfindung gemäß den Patentansprüchen wird in dem folgenden Konzept dargestellt. Dieses Konzept stellt darüber hinaus auch für sich genommen, kombiniert mit den obigen Aspekten oder mit Anspruchsgegenständen zumindest eine eigenständige Erfindung dar. Die Anmelderin behält sich vor, diese Erfindung oder Erfindungen zu einem späteren Zeitpunkt zum Gegenstand von Ansprüchen zu machen. Dies kann im Rahmen dieser Anmeldung oder auch im Rahmen von späteren Teilanmeldungen oder Nachanmeldungen unter Inanspruchnahme der Priorität dieser Anmeldung geschehen.

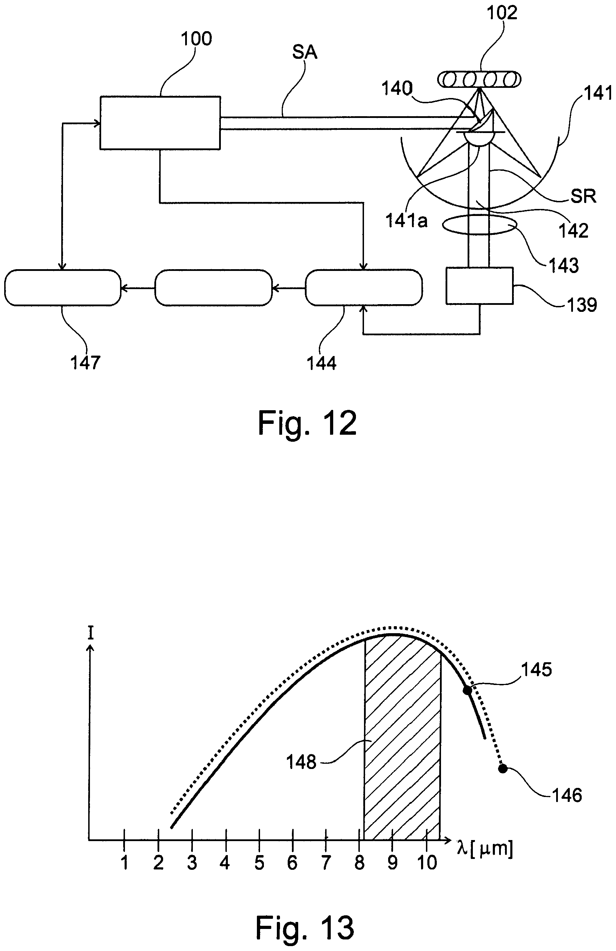

Konzept für nichtinvasive Blutzuckermessung durch eine Bestimmung der Glucose in der Haut mittels Anregung durch Quantenkaskadenlaser und Messung der Wärmewelle durch Strahlungswärme Es wird anhand der Figuren 12 und 13 ein Verfahren beschrieben, mit dem die Konzentration der Glucose oder auch einem anderen Stoff in der interstitiellen Flüssigkeit (ISF) in der Haut bestimmt werden kann. Glucose in der ISF ist repräsentativ für Blutglucose und folgt ihr schnell bei Änderungen. Das Verfahren besteht aus zumindest einzelnen oder Gruppen der folgenden Schritte oder aus der Gesamtabfolge:
1. Die Hautstelle 102 (in diesem Fall der erste Bereich der Stoffoberfläche) wird mit einem fokussierten und gegebenenfalls an einem Spiegel oder Hohlspiegel 140 reflektierten Strahl eines Quantenkaskadenlasers bestrahlt, der stufenweise oder kontinuierlich über einen bestimmten Infrarotbereich durchgestimmt wird, in dem Glucose spezifisch absorbiert. Anstelle des Quantenkaskadenlasers 100 kann auch ein Laserarray mit mehreren mit einzelnen Wellenlängen strahlenden Lasern verwendet werden. Der Spektralbereich kann (oder die einzelnen Wellenlängen, typisch 5 oder mehr Wellenlängen können) insbesondere zwischen ca. 900 und ca. 1300 cm⁻¹ liegen, in dem Glucose einen Absorptionsfingerprint , das heißt typische und repräsentative Absorptionslinien aufweist.
2. Der mit SA bezeichnete Anregungsstrahl wird kontinuierlich (CW-Laser) oder gepulst mit hoher Pulswiederholrate oder moduliert verwendet. Zusätzlich wird der Anregungsstrahl niederfrequent moduliert, insbesondere im Frequenzbereich zwischen 10 und 1000 Hz. Die niederfrequente Modulation kann mit verschiedenen periodischen Funktionen, in verschiedenen Ausführungsformen Sinus, Rechteck oder Sägezahn oder ähnlich erfolgen.
3. Durch die Bestrahlung der Haut nach dringt die IR-Strahlung bis etwa 50-100 µm tief in die Haut ein und regt - je nach Wellenlänge - bestimmte Schwingungen im Molekül Glucose an. Diese Anregungen vom Schwingungsniveau v0 nach v1 gehen innerhalb von sehr kurzer Zeit in den Grundzustand zurück; bei diesem Schritt wird Wärme frei.
4. Als Folge der Wärmeentwicklung nach (3) bildet sich eine Wärmewelle aus, die isotrop vom Ort der Absorption ausgeht. Abhängig von der thermischen Diffusionslänge, die durch die unter (2) beschriebene niederfrequenten Modulation bestimmt wird, erreicht die Wärmewelle periodisch mit der Modulationsfrequenz die Oberfläche der Haut.
5. Das periodische Auftauchen der Wärmewelle an der Oberfläche entspricht einer periodischen Modulation der Wärmestrahlungseigenschaft der Haut (Stoffoberfläche der Probe). Die Haut kann hier näherungsweise als Schwarzer Strahler beschrieben werden, dessen Gesamtemission durch das Stefan-Boltzmann Gesetz proportional zur vierten Potenz der Oberflächentemperatur ist.
6. Mit einem Detektor 139 für Wärmestrahlung, d.h. einem Infrarotdetektor, d.h. einem Thermoelement, Bolometer, Halbleiterdetektor oder ähnlichem, der auf die Hautstelle der Einstrahlung gerichtet ist, wird die unter (5) beschriebene periodische Temperaturerhöhung registriert. Sie ist abhängig von der unter (1) und (2) beschriebenen Einstrahlung von Infrarotlicht und von der unter (3) beschriebenen Absorption und somit abhängig von der Konzentration der Glucose. Die Wärmestrahlung SR (in diesem Fall das Reaktionssignal) wird mittels eines optischen Elementes, in einer Ausführungsform einer Infrarotlinse oder eines Spiegels, insbesondere eines konkaven Parabolspiegels 141, gesammelt und, in einer Ausführungsform über einen konvexen Spiegel 141a auf den Detektor 139 geleitet. Hierzu kann ein verwendeter Sammelspiegel in einer Ausführungsform eine Öffnung 142 aufweisen, durch die der gesammelte Strahl geleitet wird. Zudem kann ein Filter 143 im Strahlengang vorgesehen sein, das nur Infrarotstrahlung eines bestimmten Wellenlängenbereichs durchlässt.
7. Es kann bei der Bearbeitung der Reaktionssignale speziell die Modulationsfrequenz berücksichtigt werden, wozu das Reaktionssignal in einem Lock- in- Verstärker 144 verarbeitet werden kann. Durch Analyse der Phasenlage zwischen Anregungssignal und Wärmestrahlungssignal (Reaktionssignal) mittels einer Steuerungs- und Verarbeitungseinrichtung 147 kann die Tiefeninformation über die Tiefe unter der Stoffoberfläche gewonnen werden, aus der die Reaktionssignale vorwiegend erhalten werden.
8. Durch die Auswahl und Analyse verschiedener niederfrequenter Modulationsfrequenzen wie unter (2) beschrieben für den Anregungsstrahl und die Verknüpfung der Ergebnisse für verschiedene Modulationsfrequenzen (wobei die Ergebnisse für verschiedene Modulationsfrequenzen auch unterschiedlich gewichtet werden können) kann ebenfalls die Tiefeninformation erhalten werden. Dabei können ggf. Differenzverfahren oder andere Bestimmungsverfahren verwendet werden, um die Absorption der obersten Hautschichten zu kompensieren.
9. Um die Detektion der Wärmestrahlung nach (6) möglichst empfindlich zu machen, wird sie spektral breitbandig für den gesamten in Frage kommenden Infrarotbereich genutzt. Dabei sollen möglichst viele Bereiche der Planckschen Strahlungskurve genutzt werden. Um die Detektion für die intensive Anregungsstrahlung unempfindlich zu machen, wird die Detektion der Wärmestrahlung mit Sperrfilter (Notch-Filter) 143 für diese Anregungswellenlängen versehen. Der durch das Sperrfilter 143 durchgelassene Wellenlängenbereich 148 ist auch aus dem Diagramm der Figur 13 ersichtlich. Dort ist zudem die Intensität des Reaktionssignals wellenlängenabhängig einmal in einer ersten (durchgezogenen) Kurve 145 ohne einen Anregungsstrahl oder nur mit Anregungsstrahlung in für den nachzuweisenden Stoff unspezifischen Wellenlängen (d.h. ohne die Wellenlängen, bei denen spezifische Absorptionsbanden des Stoffes vorliegen) dargestellt und zudem in einer zweiten (gestrichelten) Kurve 146 eine vergleichbare Kurve, wobei ein Anregungsstrahl eingestrahlt wird, der spezifische Absorptionswellenlängen des nachzuweisenden Stoffes enthält.
10. Aus dem nach (6-9) gemessenen, von der Anregungswellenlänge abhängigen Wärmesignal wird somit in einer Ausführungsform, wenn Glucose nachgewiesen werden soll, zunächst bei nicht (oder unter Ausnahme von) glucoserelevanten Wellenlängen des Anregungsstrahls (Kurve 145) der Hintergrund bestimmt, anschließend bei (oder unter Einschluss von) glucoserelevanten Wellenlängen die Differenz zum Hintergrundsignal. Daraus ergibt sich die Glucosekonzentration in der Hautschicht oder den Hautschichten, die durch die ausgewählte Phasenlage nach (7) oder die verschiedenen Modulationsfrequenzen nach (8) oder deren Verknüpfung festgelegt werden.

Obwohl die Erfindung im Detail durch bevorzugte Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 10: Vorrichtung
- 100: Anregungssendeeinrichtung / Anregungs-Lichtquelle
- 100a: Emitter / Sendeelemente
- 101: Stoff
- 102: erster Bereich
- 103: Volumen
- 104: Einrichtung
- 105: Einrichtung
- 106: Detektionseinrichtung
- 107: Verarbeitungseinrichtung /Auswertungseinrichtung
- 107a: Speicher
- 108: optisches Medium
- 108a: Flächenabschnitt
- 108b: Flächenabschnitt
- 109: Justagevorrichtung
- 110: Teilfläche
- 111: Teilfläche
- 112: Messstrahl / Mess-Lichtstrahl
- 113: Spiegelfläche
- 114: Spiegelfläche
- 116: Öffnung
- 117: Öffnung
- 118: Öffnung
- 119: Anschlusskörper
- 120: Lichtwellenleiter
- 121: Träger
- 122: Gehäuse
- 123: Körper
- 124: Seite
- 125: Riemen
- 126: Fingerbeere
- 127: Justageeinrichtung
- 128: Abbildungsoptik
- 129: Abbildungsoptik
- 130: optischer Detektor / Kamera
- 131: Datenverarbeitungseinrichtung
- 132: Steuerung
- 133: Mikrospiegel
- 134: Mikrospiegel
- 135: Mikro-elektromechanisches System
- 136: Umlenkeinrichtung
- 137: Steuereinrichtung
- 138: Schicht
- 139: Infrarot- Detektor
- 140: Spiegel
- 141: Hohlspiegel
- 142: Öffnung in 141
- 143: Wellenlängenfilter
- 144: Lock-in -Verstärker
- 145: Signalkurve des Reaktionssignals (durchgezogen)
- 146: Signalkurve des Reaktionssignals (gestrichelt)
- 147: Steuerungs- und Verarbeitungseinrichtung
- 148: Wellenlängenbereich

- BZA: Blutzuckerspiegelangabe
- D: Detektionsergebnis
- GF: Grenzfläche
- SA: Anregungsstrahl
- SR: Reaktionssignal

## Patentansprüche

1. Vorrichtung (10) zum Analysieren eines Stoffes (101)
- mit einer Anregungssendeeinrichtung (100) in Form einer Laserlichtquelle zum Erzeugen mindestens eines elektromagnetischen Anregungsstrahls (SA) mit mehreren Anregungswellenlängen und
- mit einer Einrichtung (104) zur Intensitätsmodulierung des Anregungslichtstrahls (SA) sowie mit
- einer Detektionseinrichtung (106, 139) zur Detektion eines Reaktionssignals (SR),
- wobei die Vorrichtung (10) ein optisches Medium (108) aufweist, das mit dem Stoff (101) in einem ersten Bereich (102) der Oberfläche des Stoffs in direktem Kontakt steht,
- die Anregungssendeeinrichtung (100) den mindestens einen elektromagnetischen Anregungsstrahl (SA) in ein Stoffvolumen (103) einstrahlt, das unterhalb des ersten Bereiches (102) der Oberfläche des Stoffs (101) liegt,
- die Vorrichtung eine Einrichtung (105) zum Aussenden eines Messstrahls (112) umfasst, die so angeordnet ist, dass der ausgesendete Messstrahl (112) in das optische Medium (108) eindringt, welches aus einem für den Messstrahl transparenten Material , insbesondere Glas, Kristall oder einem transparenten Kunststoff besteht,
- wobei der Messlichtstrahl und der Anregungslichtstrahl auf der Grenzfläche des optischen Mediums und der Stoffoberfläche, an der der Messlichtstrahl reflektiert wird, einander unmittelbar benachbart sind oder überlappen,
- wobei die Einrichtung zum Aussenden eines Messstrahls und die Detektionseinrichtung derart zueinander ausgerichtet sind, dass die Detektionseinrichtung als das zeitabhängige Reaktionssignal den Messstrahl detektiert, nachdem dieser zumindest einmal an derjenigen Grenzfläche (GF) des optischen Mediums (108) reflektiert worden ist, die mit dem Stoff in dem ersten Bereich (102) der Oberfläche des Stoffs (101), in Kontakt steht, und
- wobei die Detektionseinrichtung (106) zur Detektion eines zeitabhängigen Reaktionssignals (SR) in Abhängigkeit von der Wellenlänge des Anregungslichts und/oder der Intensitäts-Modulation des Anregungslichts konfiguriert ist, und
- mit einer Einrichtung (107, 147) zum Analysieren des Stoffes anhand des detektierten Reaktionssignals (SR);
**dadurch gekennzeichnet, dass**
- die Anregungssendeeinrichtung (100) mehr als zwei Sendeelemente (100a) in Form eines ein-, zwei- oder mehrdimensionalen Sendeelementarrays von Lasern zur Erzeugung mindestens eines elektromagnetischen Anregungsstrahls (SA) durch den aufeinander folgenden Betrieb mehrerer Laser einer Laserlichtquelle umfasst und sich die Wellenlängen der elektromagnetischen Anregungsstrahlen der Sendeelemente (100a) unterscheiden, und
- dass die Vorrichtung so konfiguriert ist,
dass nacheinander unter Verwendung verschiedener Modulationsfrequenzen der Anregungssendeeinrichtung (100) jeweils für verschiedene Wellenlängen des Anregungsstrahls (SA) durch die Detektionseinrichtung (106) Reaktionssignale ermittelt werden und mehrere Reaktionssignalverläufe zu verschiedenen Modulationsfrequenzen miteinander verknüpft werden und dass daraus eine für einen Tiefenbereich unter der Stoffoberfläche spezifische Information gewonnen wird.

2. Vorrichtung nach Anspruch 1,
**dadurch gekennzeichnet, dass**
die zwei oder mehr Sendeelemente (100a) jeweils einen eigenen elektromagnetischen Anregungsstrahl erzeugen und diesen in das Volumen unterhalb des ersten Bereiches (102) einstrahlen.

3. Vorrichtung nach einem der voranstehenden Ansprüche,
**dadurch gekennzeichnet, dass**
die Anregungssendeeinrichtung zwei oder mehr Laser in Form eines ein-, zwei- oder mehrdimensionalen Laserarrays, und/oder zwei oder mehr Leuchtdioden in Form eines ein-, zwei- oder mehrdimensionalen Diodenarrays, umfasst.

4. Vorrichtung nach einem der voranstehenden Ansprüche,
**dadurch gekennzeichnet, dass**
die Einrichtung (104) zur Intensitätsmodulierung eine elektrische Modulationseinrichtung aufweist, die mit der Anregungssendeeinrichtung (100) elektrisch in Verbindung steht und diese elektrisch ansteuert, umfasst oder durch eine solche gebildet ist.

5. Vorrichtung nach einem der voranstehenden Ansprüche,
**dadurch gekennzeichnet, dass**
die Einrichtung (104) zur Intensitätsmodulierung wenigstens einen im Strahlengang angeordneten gesteuerten Spiegel (133, 134) umfasst.

6. Vorrichtung nach einem der voranstehenden Ansprüche,
**dadurch gekennzeichnet, dass**
die Einrichtung (104) zur Intensitätsmodulierung wenigstens eine bezüglich ihrer Transparenz steuerbare, im Strahlengang angeordnete Schicht (138) umfasst oder durch eine solche gebildet ist.

7. Vorrichtung nach einem der voranstehenden Ansprüche,
**dadurch gekennzeichnet, dass**
die Einrichtung zum Aussenden eines Messstrahls und/oder die Detektionseinrichtung und/oder Anregungssendeeinrichtung unmittelbar mit dem optischen Medium mechanisch fest verbunden und/oder mittels eines Lichtwellenleiters (120) an dieses angekoppelt ist.

8. Vorrichtung nach einem der voranstehenden Ansprüche,
**dadurch gekennzeichnet, dass**
das optische Medium unmittelbar eine Abbildungsoptik (128, 129) trägt und/oder in das optische Medium eine Abbildungsoptik (128, 129) integriert ist.

9. Vorrichtung nach einem der voranstehenden Ansprüche,
**dadurch gekennzeichnet, dass**
in oder an dem optischen Medium (108) ein oder mehrere Spiegelflächen (113, 114) zur Reflektion des Messstrahls (112) vorgesehen sind.

10. Vorrichtung nach einem der voranstehenden Ansprüche,
**dadurch gekennzeichnet, dass**
die Anregungssendeeinrichtung (100)und/oder die Einrichtung (105) zum Aussenden des Messstrahls und/oder die Detektionseinrichtung (106) unmittelbar aneinander oder an einem gemeinsamen Träger (121) befestigt sind.

11. Vorrichtung nach einem der voranstehenden Ansprüche,
**dadurch gekennzeichnet, dass**
die Anregungssendeeinrichtung einen integrierten Halbleiterbaustein umfasst, der einen oder mehrere Laserelemente sowie mindestens ein mikrooptisches Bauelement und vorzugsweise zusätzlich ein Modulationselement aufweist.

12. Verfahren zum Analysieren eines Stoffes (101) unter Verwendung einer Vorrichtung nach einem der Ansprüche 1 bis 11, wobei bei dem Verfahren
- mit der Anregungssendeeinrichtung (100) mindestens ein elektromagnetischer Anregungsstrahl (SA) mit zumindest einer Anregungswellenlänge durch den wenigstens teilweise gleichzeitigen oder aufeinander folgenden Betrieb mehrerer Laseremitter einer Laserlichtquelle erzeugt wird, die Anregungssendeeinrichtung (100) den mindestens einen elektromagnetischen Anregungsstrahl (SA) in ein Stoffvolumen (103) einstrahlt, das unterhalb eines ersten Bereiches (102) der Oberfläche des Stoffs (101) liegt,
- die Einrichtung (105) zum Aussenden eines Messstrahls (112) so angeordnet ist, dass der ausgesendete Messstrahl (112) in das optische Medium (108) eindringt, das mit dem Stoff (101) in dem ersten Bereich (102) der Oberfläche des Stoffs in direktem Kontakt steht und
- mit einer Detektionseinrichtung (106), ein Reaktionssignal (SR) detektiert wird, wobei die Einrichtung zum Aussenden eines Messstrahls und die Detektionseinrichtung derart zueinander ausgerichtet sind, dass die Detektionseinrichtung als das zeitabhängige Reaktionssignal den Messstrahl detektiert, nachdem dieser zumindest einmal an derjenigen Grenzfläche (GF) des optischen Mediums (108) reflektiert worden ist, die mit dem Stoff in dem ersten Bereich (102) der Oberfläche des Stoffs (101) in Kontakt steht und
- anhand des detektierten Reaktionssignals (SR) der Stoff analysiert wird, wobei
- nacheinander unter Verwendung verschiedener Modulationsfrequenzen der Anregungssendeeinrichtung Reaktionssignale, insbesondere zeitliche Reaktionssignalverläufe für verschiedene Wellenlängen des Anregungsstrahls ermittelt und
- mehrere Reaktionssignalverläufe zu verschiedenen Modulationsfrequenzen miteinander verknüpft werden und wobei
- daraus insbesondere eine für einen Tiefenbereich unter der Stoffoberfläche spezifische Information gewonnen wird.

13. Verfahren nach Anspruch 12,
**dadurch gekennzeichnet, dass**
- bei Verwendung von mehreren Modulationsfrequenzen des Pumpstrahls zur gleichen Zeit das detektierte Signal mittels eines Analyseverfahrens, vorzugsweise einer Fourier-Transformation, seinen Frequenzen entsprechend getrennt wird und
- jeweils nur das Teilsignal herausgefiltert wird, das der gewünschten Frequenz entspricht.

14. Verfahren nach einem der voranstehenden Ansprüche 12-13,
**dadurch gekennzeichnet, dass**
- ein optisches Medium (108) mit dem Stoff (101), insbesondere einem ersten Bereich (102) der Oberfläche des Stoffs (101), in direkten Kontakt gebracht wird,
- mit der Anregungssendeeinrichtung (100) der ausgesendete Anregungsstrahl (SA) erzeugt und insbesondere derart ausgestrahlt wird, dass dieser in das optische Medium (108) eindringt und dieses an einem vorbestimmten Punkt an der Oberfläche des optischen Mediums (108) wieder verlässt,
- mit einer Einrichtung (105) zum Aussenden eines Messstrahls (112) ein Messstrahl (112), insbesondere Mess-Lichtstrahl, derart erzeugt wird, dass dieser in das optische Medium (108) eindringt und dass insbesondere der Messstrahl (112) und der Anregungsstrahl (SA) sich im Betrieb auf einer Grenzfläche (GF) des optischen Mediums (108) und der Oberfläche des Stoffs (101), an der der Messstrahl (112) reflektiert wird, überlappen, und
- mit der Detektionseinrichtung (106) ein das Reaktionssignal (SR) bildender reflektierter Messstrahl (112) gemessen
- und/oder direkt oder indirekt die Ablenkung des reflektierten Messstrahls detektiert wird.

15. Verfahren nach einem der voranstehenden Ansprüche 12-14,
**dadurch gekennzeichnet, dass**
in Abhängigkeit von einer in dem Stoff ermittelten Stoffkonzentration eine Dosiereinrichtung zur Abgabe einer Substanz in den Stoff, insbesondere in einen Patientenkörper angesteuert wird und/oder ein akustisches und/oder optisches Signal ausgegeben wird und/oder ein Signal mittels einer Funkverbindung an eine Verarbeitungseinrichtung abgegeben wird.

## Claims

1. A device (10) for analyzing a material (101)
- having an excitation transmission device (100) formed by a laser light source for generating at least one electromagnetic excitation light beam (SA) with plural excitation wave lengths and
- having a device (104) for modulating the intensity of the excitation light beam (SA) and
- having a detection device (106, 139) for detecting a response signal (SR),
- wherein the device (10) comprises an optical medium (108), which is in direct contact with the material (101) in a first region (102) of the surface of the material,
- wherein the excitation transmission device (100) emits an electromagnetic excitation beam (SA) in a material volume (103) which lies beneath the first region (102) of the surface of the material (101),
- wherein the device comprises a system (105) for emitting a measurement beam (112) which is arranged such that the emitted measurement beam (112) penetrates into the optical medium (108), which is made from a material that is transparent for the measurement beam, and in particular from glass, crystal or a transparent plastic,
- wherein the measurement light beam and the excitation light beam are adjacent to each other or overlapping on the interface between the optical medium and the material surface,
- wherein the system for emitting a measurement beam and the detection device are arranged with respect to each other such that the detection device detects the measurement beam as said time dependent response signal, after the measurement beam has been reflected at least once at the interface (GF) of the optical medium (108), which is in contact with the material in the first region (102) of the surface of the material (101), and
- wherein the detection device (106) is configured for detecting a time dependent response signal (SR) as a function of the wavelength of the excitation light and/or of the intensity modulation of the excitation light, and
- having a device (107, 147) for analyzing the material based on the detected response signal (SR);
**characterized in that**
- the excitation transmission device (100) comprises more than two transmission elements (100a) in the form of a one, two or multidimensional array of transmission elements of lasers for generating at least one electromagnetic excitation beam (SA) by means of successively operating plural lasers of a laser light source and **in that** the wavelengths of the electromagnetic excitation beams of the transmission elements (100a) are different from each other, and
- **in that** the device is configured such that response signals are subsequently determined, by the detection device (106) using different modulation frequencies of the excitation transmission device (100) each for different wavelengths of the excitation beam (SA), and plural response signal waveforms or patterns corresponding to different modulation frequencies are combined with each other and that from this information specific to a depth region beneath the material surface is obtained.

2. Device according to claim 1, **characterized in that** the two or more transmission elements (100a) each generate an electromagnetic excitation beam of their own and emit the same into the volume beneath the first region (102).

3. The Device according to one of the preceding claims, **characterized in that** the excitation transmission device comprises two or more lasers in the form of a one, two or multidimensional laser array and/or two or more light emitting diodes in the form of a one, two or multidimensional diode array.

4. The device according to any one of the preceding claims, **characterized in that** the device (104) for the intensity modulation comprises or is formed by an electrical modulation device, which is electrically connected to the excitation transmission device (100) and electrically controls it.

5. The device according to any one of the preceding claims, **characterized in that** the device (104) for intensity modulation comprises at least one controlled mirror (133, 134) arranged in the beam path.

6. The device according to any one of the preceding claims, **characterized in that** the device (104) for intensity modulation comprises at least one layer which is arranged in the beam path (138) and is controllable with respect to its transparency, or is formed by such a layer.

7. The device according to any one of the preceding claims, **characterized in that** the device for emitting a measuring beam and/or the detection device and/or the excitation transmission device are directly mechanically fixedly connected to the optical medium and/or coupled to the same by means of a fibre-optic cable (120).

8. The device according to any one of the preceding claims, **characterized in that** the optical medium directly supports an imaging optics (128, 129), and/or an imaging optics (128, 129) is integrated into the optical medium.

9. The device according to any one of the preceding claims, **characterized in that** one or more reflective surfaces (113, 114) are provided in or on the optical medium (108) for reflecting the measuring beam (112).

10. The device according to any one of the preceding claims, **characterized in that** the excitation transmission device (100) and/or the device (105) for the emission of the measuring beam and/or the detection device (106) are directly attached to each other or to a common support (121).

11. The device according to any one of the preceding claims, **characterized in that** the excitation transmission device has an integrated semiconductor component, which comprises one or more laser elements and at least one micro-optical component and preferably additionally a modulation element.

12. A method for analyzing a material (101) using a device according to one of claims 1 to 11, wherein in said method
- using the excitation transmission device (100), at least one electromagnetic excitation beam (SA) having at least one excitation wavelength is generated by means of the at least in part simultaneous or successive operation of plural laser emitters of a laser light source, and the excitation transmission device (100) emits the at least one electromagnetic excitation beam (SA) in a material volume (103) which lies beneath a first region (102) of the surface of the material (101),
- wherein the system (105) for emitting a measurement beam (112) is arranged such that the emitted measurement beam (112) penetrates into the optical medium (108), which is in direct contact with the material (101) in the first region (102) of the surface of the material, and
- wherein a response signal (SR) is detected using a detection device (106), wherein the system for emitting a measurement beam and the detection device are arranged with respect to each other such that the detection device detects the measurement beam as said time dependent response signal, after the measurement beam has been reflected at least once at the interface (GF) of the optical medium (108) which is in contact with the material in the first region (102) of the surface of the material (101), and
- the material is analyzed based on the detected response signal (SR), wherein
- response signals, in particular response signal wave forms or patterns for different wavelengths of the excitation beam are determined successively using different modulation frequencies of the excitation transmission device and
- plural response signal waveforms or patterns for different modulation frequencies are combined with each other, and wherein
- from this in particular information is obtained which is specific for a depth region beneath the material surface.

13. The method according to Claim 12,
**characterized in that**
- when a plurality of modulation frequencies of the pump beam are used at the same time, the detected signal is resolved into its frequencies by means of an analytical procedure, preferably a Fourier transform, and
- only the partial signal that corresponds to the desired frequency is filtered out.

14. The method according to any one of the preceding Claims 12 to 13, **characterized in that**
- an optical medium (108) is brought into direct contact with the material (101), in particular with a first region (102) of the surface of the material (101),
- the emitted excitation beam (SA) is generated with the excitation transmission device (100) and, in particular, radiated in such a way that it penetrates the optical medium (108) and exits the same at a predetermined point on the surface of the optical medium (108),
- with a device (105) for emitting a measuring beam (112), a measuring beam (112), in particular a measuring light beam, is generated in such a way that it penetrates the optical medium (108) and that in particular, in operation the measuring beam (112) and the excitation beam (SA) overlap at an interface (GF) of the optical medium (108) and the surface of the material (101), at which the measuring beam (112) is reflected, and
- a reflected measuring beam (112) forming the response signal (SR) is measured with the detection device (106),
- and/or the deflection of the reflected beam is directly or indirectly detected.

15. The method according to any one of the preceding Claims 12 to 14, **characterized in that** as a function of a material concentration identified in the material, a dosing device is activated for delivering a substance into the material, in particular into a body of a patient, and/or an acoustic and/or visual signal is output and/or a signal is delivered to a processing device via a wireless connection.

## Revendications

1. Dispositif (10) pour l'analyse d'un matériau (101)
- avec un dispositif d'envoi d'excitation (100) sous la forme d'une source de lumière laser pour la production d'au moins un rayon d'excitation électromagnétique (SA) avec plusieurs longueurs d'ondes d'excitation et
- avec un système (104) pour la modulation d'intensité du rayon d'excitation (SA) ainsi qu'avec
- un dispositif de détection (109, 139) pour la détection d'un signal de réaction (SR),
- le dispositif (10) comprenant un milieu optique (108) qui est en contact directe avec le matériau (101) dans une première zone (102) de la surface du matériau,
- le dispositif d'envoi d'excitation (100) émettant l'au moins un rayon d'excitation (SA) dans un volume de matériau (103) qui se trouve en dessous de la première zone (102) de la surface du matériau (101),
- le dispositif comprenant un système (105) pour l'émission d'un rayon de mesure (112), qui est disposé de façon à ce que le rayon de mesure (112) émis pénètre dans le milieu optique (108) qui est constitué d'un matériau transparent pour le rayon de mesure, plus particulièrement du verre, du cristal ou une matière plastique transparente,
- le rayon lumineux de mesure et le rayon lumineux d'excitation étant directement adjacents entre eux ou se superposant sur la surface limite du milieu optique et de la surface du matériau, sur laquelle le rayon lumineux de mesure est réfléchi,
- le système d'émission d'un rayon de mesure et le dispositif de détection étant orientés l'un par rapport à l'autre de façon à ce que le dispositif de détection détecte le rayon de mesure en tant que signal de réaction en fonction du temps, après que celui-ci ait été réfléchi au moins une fois au niveau de la surface limite (GF) du milieu optique (108), qui est en contact avec le matériau dans la première zone (102) de la surface du matériau (101) et
- le dispositif de détection (106) est conçu pour la détection d'un signal de réaction (SR) dépendant du temps en fonction de la longueur d'onde de la lumière d'excitation et/ou de la modulation d'intensité de la lumière d'excitation et
- avec un système (107, 407) pour l'analyse du matériau à l'aide du signal de réaction (SR) détecté ;
**caractérisé en ce que**
- le dispositif d'envoi d'excitation (100) comprend plus de deux éléments émetteurs (100a) sous la forme d'une matrice d'éléments émetteurs mono-, bi- ou pluridimensionnels de lasers pour la production d'au moins un rayon d'excitation électromagnétique (SA) grâce au fonctionnement successif de plusieurs lasers d'une source de lumière laser et les longueurs d'ondes des rayons d'excitation électromagnétiques des éléments émetteurs (100a) sont différentes et
- le dispositif est conçu de façon à ce que
des signaux de réaction soient déterminés successivement à l'aide de différentes fréquences de modulation du dispositif d'envoi d'excitation (100) pour différentes longueurs d'ondes du rayon d'excitation (SA) par le dispositif de détection (106) et plusieurs tracés de signaux de réaction sont combinées entre eux en différentes fréquences de modulation et à ce que des informations spécifiques soient obtenues pour une zone de profondeur sous la surface du matériau.

2. Dispositif selon la revendication 1,
**caractérisé en ce que**
deux éléments émetteurs (100a) ou plus émettent chacun leur propre rayon d'excitation électromagnétique et irradient celui-ci dans le volume en dessous de la première zone (102).

3. Dispositif selon l'une des revendications précédentes,
**caractérisé en ce que**
le dispositif d'envoi d'excitation comprend deux lasers ou plus sous la forme d'une matrice de lasers mono-, bi- ou pluridimensionnelle et/ou deux diodes électroluminescentes ou plus sous la forme d'une matrice de diodes mono-, bi- ou pluridimensionnelle.

4. Dispositif selon l'une des revendications précédentes,
**caractérisé en ce que**
le système (104) pour la modulation d'intensité comprend ou est constitué d'un dispositif de modulation électrique qui est relié électriquement avec le dispositif d'envoi d'excitation (100) et qui contrôle celui-ci de manière électrique.

5. Dispositif selon l'une des revendications précédentes,
**caractérisé en ce que**
le système (104) pour la modulation d'intensité comprend au moins un miroir contrôlé (133, 134) disposé dans le trajet des rayons.

6. Dispositif selon l'une des revendications précédentes,
**caractérisé en ce que**
le système (104) pour la modulation d'intensité comprend ou est constitué d'au moins une couche (138) dont la transparence peut être contrôlée et disposée dans le trajet des rayons.

7. Dispositif selon l'une des revendications précédentes,
**caractérisé en ce que**
le système pour l'émission d'un rayon de mesure et/ou le dispositif de détection et/ou le dispositif d'envoi d'excitation est relié mécaniquement fermement avec le milieu optique et/ou est couplé à celui-ci au moyen d'une fibre optique (120).

8. Dispositif selon l'une des revendications précédentes,
**caractérisé en ce que**
le milieu optique supporte directement une optique de reproduction (128, 129) et/ou une optique de reproduction (128, 129) est intégrée dans le milieu optique.

9. Dispositif selon l'une des revendications précédentes,
**caractérisé en ce que**
dans ou sur le milieu optique (108) sont prévus un ou plusieurs surfaces réfléchissantes (113, 114) pour la réflexion du rayon de mesure (112).

10. Dispositif selon l'une des revendications précédentes,
**caractérisé en ce que**
le dispositif d'envoi d'excitation (100) et/ou le système (105) pour l'émission du rayon de mesure et/ou le dispositif de détection (106) sont fixés directement l'un à l'autre ou à un support commun (121).

11. Dispositif selon l'une des revendications précédentes,
**caractérisé en ce que**
le dispositif d'envoi d'excitation comprend un composant semi-conducteur intégré qui comprend un ou plusieurs éléments laser ainsi qu'au moins un composant micro-optique et de préférence en outre un élément de modulation.

12. Procédé d'analyse d'un matériau (101) à l'aide d'un dispositif selon l'une des revendications 1 à 11, dans lequel
- avec le dispositif d'envoi d'excitation (100), au moins un rayon d'excitation électromagnétique (SA) avec au moins une longueur d'onde d'excitation est produit par le fonctionnement au moins partiellement simultané ou successif de plusieurs émetteurs lasers d'une source de lumière laser, le dispositif d'envoi d'excitation (100) émettant l'au moins un rayon d'excitation électromagnétique (SA) dans un volume de matériau (103) qui se trouve en dessous d'une première zone (102) de la surface du matériau (101),
- le système (105) d'émission d'un rayon de mesure (112) est disposé de façon à ce que le rayon de mesure (112) émis pénètre dans le milieu optique (108) qio est en contact direct avec le matériau (101) dans la première zone (102) de la surface du matériau et
- avec un dispositif de détection (106), un signal de réaction (SR) est détecté, le système d'émission d'un rayon de mesure et le dispositif de détection étant orientés l'un par rapport à l'autre de façon à ce que le dispositif de détection détecte le rayon de mesure en tant que signal de réaction dépendant du temps, après que celui-ci ait été réfléchi au moins une fois au niveau de la surface limite (GF) du milieu optique (108) qui est en contact avec le matériau dans la première zone (102) de la surface du matériau (101) et
- à l'aide du signal de réaction (SR) détecté, le matériau est analysée,
- des signaux de réaction étant déterminés successivement à l'aide de différentes fréquences de modulation du dispositif d'envoi d'excitation, plus particulièrement des tracés de signaux de réaction temporels pour différentes longueurs d'ondes sont déterminés pour différentes longueurs d'ondes du rayon d'excitation et
- plusieurs tracés de signaux de réaction étant combinés entre eux en différentes fréquences de modulation et
- des informations spécifiques en étant déduites pour une zone de profondeur sous la surface du matériau.

13. Procédé selon la revendication 12,
**caractérisé en ce que**
- à l'aide de plusieurs fréquences de modulation du rayon de pompage, le signal détecté est simultanément séparé en fonction de ses fréquences au moyen d'un procédé d'analyse, de préférence d'une transformation de Fourier et
- seul le signal partiel correspondant à la fréquence souhaitée est extrait.

14. Procédé selon l'une des revendications précédentes 12 à 13,
**caractérisé en ce que**
- un milieu optique (108) est mis en contact direct avec le matériau (101), plus particulièrement une première zone (102) de la surface du matériau (101),
- avec le dispositif d'envoi d'excitation (100), le rayon d'excitation (SA) émis est produit et plus particulièrement de façon à ce qu'il pénètre dans le milieu optique (108) et quitte à nouveau celui-ci au niveau d'un point prédéterminé à la surface du milieu optique (108),
- avec un système (105) pour l'émission d'un rayon de mesure (112), un rayon de mesure (112), plus particulièrement un rayon lumineux de mesure, est produit de façon à ce qu'il pénètre dans le milieu optique (108) et à ce que plus particulièrement le rayon de mesure (112) et le rayon d'excitation (SA) se superposent, lors du fonctionnement, sur une surface limite (GF) du milieu optique (108) et de la surface du matériau (101), au niveau de laquelle le rayon de mesure (112) est réfléchi et
- avec le dispositif de détection (106), un rayon de mesure (112) formant le signal de réaction (SR) est mesuré
- et/ou la déviation du rayon de mesure réfléchi est détectée directement ou indirectement.

15. Procédé selon l'une des revendications précédentes 12 à 14,
**caractérisé en ce que**
en fonction d'une concentration déterminée dans le matériau, un dispositif de dosage pour la distribution d'une substance dans le matériau, plus particulièrement dans le corps d'un patient est contrôlée et/ou un signal acoustique et/ou optique est émis et/ou un signal est transmis au moyen d'une liaison radio à un dispositif de traitement.
